# DEMANDE DE BREVET EUROPEEN

(11) **EP 4 817 556 A2**
(43) Date de publication de la demande: **07.10.2026**
(21) Numéro de dépôt: 26200153.0
(22) Date de dépôt: 16.02.2022
(51) Int. Cl.: A61C 9/00

(54) **KIT D'ACQUISITION**

(30) Priorité: 17.02.2021 FR 2101517
(62) Numéro(s) de document de la (des) demande(s) initiale(s) en application de l'article 76 CBE: 22706580.2 / 4 294 249
(71) Demandeur: Dental Monitoring, 75017 Paris (FR)
(72) Inventeur: PELLISSARD, Thomas, 94700 MAISONS-ALFORT (FR); LANCON, Cedric, 39600 VILLERS FARLAY (FR); ROISIN, Louis-Charles, 75016 PARIS (FR); SALAH, Philippe, 75017 PARIS (FR)
(74) Mandataire: Ipsilon

(57) **Abrégé**

Kit d'acquisition comportant :
- un téléphone portable (12) ;
- un support (14) de téléphone portable comportant
- un espaceur (24) débouchant par une ouverture orale (Oo) destinée à être introduite dans la bouche de l'utilisateur et par une ouverture d'acquisition (Oa'), et
- un adaptateur (26) fixé sur l'espaceur (24) et définissant avec l'espaceur (24) une chambre (28) débouchant par l'ouverture orale (Oo) et par une ouverture d'adaptateur (Oa), l'adaptateur comportant une embase (50) sur laquelle le téléphone portable est rigidement fixé, de manière amovible, dans une position d'acquisition dans laquelle l'objectif (16) du téléphone portable a une vue au moins partielle de l'ouverture orale (Oo), à travers l'ouverture d'adaptateur (Oa) et l'ouverture d'acquisition (Oa'),

l'adaptateur (26) comportant des premier et deuxième mors (52₁ ; 52₂) entre lesquels le téléphone portable est serré, suivant une direction de serrage (Z₅₂).

## Description

### Domaine technique

La présente invention concerne un kit d'acquisition, et en particulier de photos dentaires, en particulier pour la mise en œuvre d'un procédé tel que décrit dans la demande internationale PCT/EP2015/074896.

### Etat de la technique

PCT/EP2015/074897 décrit un kit d'acquisition permettant d'acquérir, au moyen d'un téléphone portable, des photos extraorales des dents d'un utilisateur, bouche fermée ou bouche ouverte.

Il existe un besoin permanent pour un kit d'acquisition
- peu encombrant,
- peu couteux à fabriquer,
- de préférence réutilisable,
- qui puisse être facilement manipulé et mis en position de service dans la bouche de l'utilisateur,
- puis qui permette, avec le téléphone portable, l'acquisition facile de données 2D ou 3D par l'utilisateur lui-même, en vues occlusales ou latérales, bouche ouverte ou bouche fermée,
dans des conditions d'hygiène optimales.

Les données 2D ou 3D, relatives aux dents, peuvent être en particulier des images extraorales ou des modèles numériques tridimensionnels.

Le kit doit permettre l'acquisition de données pour chaque dent de l'utilisateur, et en particulier de dents au fond de sa bouche.

Un objectif de la présente invention est de répondre, au moins partiellement, à ce besoin.

### Résumé de l'invention

L'invention propose un kit d'acquisition comportant :
- un téléphone portable comportant
   - une face avant,
   - une face arrière présentant un écran, et
   - un chant, définissant l'épaisseur du téléphone portable et joignant les faces avant et arrière ;
   le téléphone portable étant muni d'un objectif d'acquisition de données 2D et/ou 3D, de préférence disposé sur la face avant ;
- un support de téléphone portable comportant
   - un espaceur débouchant par une ouverture orale destinée à être introduite dans la bouche de l'utilisateur et par une ouverture d'acquisition, et
   - un adaptateur, de préférence de forme générale tubulaire, fixé sur l'espaceur et définissant avec l'espaceur une chambre débouchant par l'ouverture orale et par une ouverture d'adaptateur, l'ouverture d'adaptateur pouvant être confondue avec l'ouverture d'acquisition, l'adaptateur comportant une embase sur laquelle le téléphone portable est rigidement fixé, de préférence de manière amovible, dans une position d'acquisition dans laquelle l'objectif a une vue au moins partielle de l'ouverture orale, à travers l'ouverture d'adaptateur et l'ouverture d'acquisition.

Après plusieurs années de recherche, les inventeurs ont découvert de nombreuses caractéristiques avantageuses pour un tel kit d'acquisition.

Un tel kit d'acquisition peut en particulier comporter une ou plusieurs des caractéristiques préférées suivantes :
**Selon un premier aspect principal, particulièrement avantageux pour améliorer l'interfaçage avec** l'utilisateur :
- le téléphone portable est fixé sur l'embase de manière que moins de 20%, de préférence moins de 15%, de préférence moins de 10%, de préférence moins de 5%, de préférence moins de 2 % de l'aire de la face arrière soient masqués par l'adaptateur ;
- la face arrière du téléphone portable n'est pas en contact avec l'adaptateur ou l'aire de la surface de contact entre la face arrière du téléphone portable et l'adaptateur représente moins de 40%, de préférence moins de 30%, de préférence moins de 20%, de préférence moins de 10%, de préférence moins de 5 % de l'aire de ladite face arrière ;
- le téléphone portable est serré selon une direction de serrage, de préférence serré perpendiculairement à la direction de la longueur ou de la largeur du téléphone portable, de préférence parallèlement à la face avant du téléphone portable, contre l'embase de l'adaptateur ;
- le téléphone portable est serré contre l'embase en une pluralité de points de serrage, la plus grande distance entre des points de serrage, mesurée perpendiculairement à la direction de serrage, de préférence mesurée suivant la direction de la longueur du téléphone portable étant supérieure à 1 cm, de préférence supérieure à 2 cm, de préférence supérieure à 3 cm, de préférence supérieure à 4 cm, et/ou inférieure à 10 cm ;
- l'adaptateur comporte au moins une sangle, de préférence élastique, et/ou au moins un premier mors pressant le téléphone portable contre l'embase ;
- l'ouverture d'adaptateur est latéralement décalée par rapport à la zone de serrage du téléphone portable sur l'adaptateur,
   un décalage latéral étant une distance non nulle, suivant une direction de décalage perpendiculaire à la direction de serrage et dans un plan parallèle au plan de la face avant du téléphone portable, entre la position du centre de l'ouverture d'adaptateur et la position du centre de la zone de serrage ;
- le décalage latéral est supérieur à 3 cm et inférieur à 10 cm.

**Selon un deuxième aspect principal, particulièrement avantageux pour l'assemblage du téléphone portable** :
- l'adaptateur, en particulier l'embase, comporte des premier et deuxième mors entre lesquels le téléphone portable est serré, suivant une direction de serrage ;
- la direction de serrage est de préférence perpendiculaire à la direction de la longueur du téléphone portable et/ou perpendiculaire à la direction de la largeur du téléphone portable et/ou suivant une diagonale joignant deux coins du téléphone portable ;
- l'adaptateur est dépourvu de butée limitant le déplacement perpendiculairement à la direction de serrage ;
- le premier mors et/ou le deuxième mors présente(nt) la forme d'un berceau recevant le chant du téléphone portable.
- le premier mors et/ou le deuxième mors est mobile sur l'embase de manière incrémentielle ;
- l'adaptateur comporte :
   - un premier système anti-retour désactivable empêchant un mouvement du premier mors et/ou du deuxième mors à l'écart du téléphone portable, dit « mouvement d'écartement », et
   - un organe élastique, par exemple un ressort, agissant sur le premier mors et/ou le deuxième mors, respectivement, de manière à produire un dit mouvement d'écartement ;
- le premier système anti-retour comporte :
   - une roue à rochet mécaniquement couplée au premier mors et/ou au deuxième mors de manière qu'une rotation de la roue à rochet entraine un déplacement du premier mors et/ou du deuxième mors par rapport à l'embase,
   - un premier cliquet désactivable, engagé dans la roue à rochet, et
   - un premier organe élastique, par exemple un ressort, poussant élastiquement le premier cliquet contre la roue à rochet ;
- le premier mors et/ou le deuxième mors comporte une première crémaillère, et l'adaptateur comporte une roue étagée montée à rotation sur l'embase, et comportant
   - la roue à rochet, et
   - un pignon, coaxial à la roue à rochet, coopérant avec la première crémaillère de manière qu'une rotation du pignon entraine le premier mors et/ou le deuxième mors, respectivement ;
- le premier mors et/ou le deuxième mors comporte(nt) un tampon élastiquement déformé et en appui sur le téléphone portable, la déformabilité du tampon autorisant au moins un passage entre deux positions indexées consécutives définies par la coopération de la roue à rochet et du premier cliquet.

**Selon un troisième aspect principal, particulièrement avantageux pour l'utilisation d'un téléphone portable quelconque** :
- l'adaptateur comporte une platine montée mobile sur l'embase et sur laquelle l'espaceur est rigidement fixé, de manière amovible, par exemple par serrage, ou non amovible, par exemple en formant un ensemble monobloc ;
- l'espaceur est de préférence monté sur l'embase par clipsage ;
- la platine est montée coulissante sur l'embase suivant au moins une direction, de préférence exclusivement suivant une unique direction de coulissement ;
- de préférence, le téléphone portable est serré sur l'embase suivant une direction de serrage ;
- la direction de coulissement est parallèle à la direction de serrage ;
- l'adaptateur comporte un verrou désactivable permettant de sélectivement bloquer en position la platine par rapport à l'embase ;
- la platine est mobile sur l'embase de manière incrémentielle ;
- l'adaptateur comporte un deuxième système anti-retour désactivable empêchant un mouvement de retour de la platine par rapport à l'embase ;
- le deuxième système anti-retour comporte :
   - une deuxième crémaillère,
   - un deuxième cliquet désactivable, engagé dans la deuxième crémaillère, et
   - un deuxième organe élastique, par exemple un ressort, poussant élastiquement le deuxième cliquet contre la deuxième crémaillère ;
- l'ouverture d'acquisition est latéralement décalée par rapport à la zone de serrage du téléphone portable sur l'adaptateur,
   un décalage latéral étant une distance non nulle, suivant une direction de décalage perpendiculaire à la direction de serrage et dans un plan parallèle au plan de la face avant du téléphone portable, entre la position du centre de l'ouverture d'acquisition et la position du centre de la zone de serrage ;
- le décalage latéral est supérieur à 1 cm, de préférence supérieur à 2 cm, de préférence supérieur à 3 cm, de préférence supérieur à 4 cm, et/ou inférieur à 10 cm.

**Selon un quatrième aspect principal, particulièrement avantageux pour améliorer l'hygiène :**
- l'espaceur peut être venu de matière avec l'embase ou l'adaptateur peut être fixé de manière amovible sur l'espaceur ;
- si l'adaptateur est fixé de manière amovible sur l'espaceur, de préférence, l'adaptateur comporte une partie d'attache d'adaptateur et l'espaceur comporte une partie d'attache d'espaceur coopérant, dans ladite position d'acquisition, avec la partie d'attache d'adaptateur pour fixer l'adaptateur sur l'espaceur, la partie d'attache d'espaceur étant configurée pour conserver sa forme lorsqu'elle est accouplée et/ou désaccouplée de la partie d'attache d'adaptateur ;
- de préférence, aucun point de la surface de la partie d'attache d'espaceur ne se déplace, lors dudit accouplement et/ou dudit désaccouplement, de plus de 1 mm, de préférence de plus de 0,5 mm, de préférence de plus de 0,2 mm ;
- la partie d'attache d'espaceur est formée par un renfoncement et/ou un bourrelet de matière de l'espaceur ;
- la distance entre la partie d'attache d'espaceur et l'ouverture d'acquisition est inférieure à 3 cm, de préférence inférieure à 2 cm, de préférence inférieure à 1 cm, de préférence inférieure à 0,5 cm.

**Selon un cinquième aspect principal, particulièrement avantageux pour la préservation de l'environnement :**
- l'espaceur est en un matériau biodégradable, de préférence en une matière fibreuse, de préférence cellulosique, de préférence en carton.

**Selon un sixième aspect principal, particulièrement avantageux pour améliorer l'hygiène et l'observance :**
- l'espaceur et/ou l'adaptateur, de préférence l'espaceur, porte une marque de personnalisation, de préférence relative à l'utilisateur et/ou à une pathologie de l'utilisateur et/ou à un professionnel de soins dentaires et/ou à un groupement de professionnels de soins dentaires ;
- la marque de personnalisation comporte un identifiant de l'utilisateur et/ou de la pathologie de l'utilisateur et/ou du professionnel de soins dentaires et/ou du groupement de professionnels de soins dentaires ;
- la marque de personnalisation est fixée au moyen d'un ou plusieurs clip(s), vis, bande(s) auto agrippante(s) de type Velcro^{®}, bande(s) élastique(s), aimant(s), crochet(s), verrou(s), sangle(s), ventouse(s), ou d'un matériau adhésif, ou est inscrite, ou gravée ;
- la marque de personnalisation est amovible ou effaçable ;
- la marque de personnalisation est portée par un autocollant ou un aimant ou un jeton clipsé ;
- la marque de personnalisation représente un dessin ou une marque commerciale ou une forme tridimensionnelle ;
- la marque de personnalisation est en une couleur et/ou en un matériau différent de la couleur et/ou du matériau, respectivement, de l'espaceur et/ou l'adaptateur qui la porte(nt) ;
- la marque de personnalisation est portée par l'espaceur, l'espaceur étant de préférence un matériau fibreux, de préférence en carton.

**Selon un septième aspect principal, particulièrement avantageux pour améliorer l'hygiène et la préservation de l'environnement :**
- l'adaptateur est fixé de manière amovible sur l'espaceur ;
- le kit d'acquisition comporte, outre l'espaceur fixé à l'adaptateur, au moins un autre espaceur assemblable à l'adaptateur et différent de l'espaceur fixé à l'adaptateur ;
- ledit autre espaceur diffère de l'espaceur fixé à l'adaptateur en ce qu'il porte une marque de personnalisation différente et/ou présente une ouverture orale différente et/ou présente une longueur différente ;
- de préférence, ledit autre espaceur diffère de l'espaceur fixé à l'adaptateur en ce qu'il porte une marque de personnalisation différente et/ou présente une ouverture orale différente, mais présente une longueur identique, ce qui, avantageusement, permet une configuration d'acquisition du téléphone portable identique quel que soit l'espaceur utilisé.

**Selon un huitième aspect principal, particulièrement avantageux pour améliorer les conditions d'acquisition :**
- dans ladite position d'acquisition, l'objectif et le flash sont face à l'ouverture d'adaptateur et/ou face à l'ouverture d'acquisition ;
- ladite chambre ne contient aucune source lumineuse artificielle ;
- le kit d'acquisition ne comporte que le flash pour éclairer l'ouverture orale, c'est-à-dire que le flash est l'unique source lumineuse artificielle pour éclairer l'ouverture orale ;
- la plus petite dimension de l'ouverture d'adaptateur et/ou de l'ouverture d'acquisition, par exemple la largeur pour une ouverture de forme rectangulaire, est supérieure à 1 cm, de préférence supérieure à 2 cm, de préférence supérieure à 3 cm, et/ou inférieure à 5 cm, de préférence inférieure à 4 cm ;
- la plus grande dimension de l'ouverture d'adaptateur et/ou l'ouverture d'acquisition, par exemple la diagonale pour une ouverture de forme rectangulaire, est supérieure à 2 cm, de préférence supérieure à 3 cm, de préférence supérieure à 4 cm, de préférence supérieure à 5 cm, et/ou inférieure à 10 cm, de préférence inférieure à 9 cm, de préférence inférieure à 8 cm, de préférence inférieure à 7 cm, de préférence inférieure à 6 cm ;
- l'ouverture d'adaptateur et/ou l'ouverture d'acquisition a/ont une forme générale rectangulaire, dont les angles sont de préférence arrondis, ce qui facilite le nettoyage ;
- l'ouverture d'adaptateur et l'ouverture d'acquisition ont des aires qui différent de moins de 20%, de préférence de moins de 10%, de préférence de moins de 5%, de préférence de moins de 1% l'une de l'autre, sur la base de l'aire de l'ouverture d'adaptateur ;
- de préférence, les aires de l'ouverture d'adaptateur et de l'ouverture d'acquisition sont identiques, lesdites ouvertures étant de préférence confondues ;
- l'aire de l'ouverture d'adaptateur est inférieure ou supérieure à l'aire de l'ouverture orale ;
- l'ouverture d'adaptateur et l'ouverture orale ont des aires qui différent de moins de 30%, de moins de 20%, et/ou de plus de 5%, de préférence de plus de 10% l'une de l'autre, sur la base de l'aire de l'ouverture d'adaptateur ;
- le kit comporte un premier espaceur comportant une première ouverture orale présentant une aire supérieure, de préférence de plus de 10% et/ou de moins de 30%, à l'aire de l'ouverture d'adaptateur, et un deuxième espaceur comportant une deuxième ouverture orale présentant une aire inférieure, de préférence de plus de 10% et/ou de moins de 30%, à l'aire de l'ouverture d'adaptateur, chacun des premier et deuxième espaceurs pouvant être monté, de manière amovible sur l'adaptateur ;
- les premier et deuxième espaceurs comportent des premier et deuxième pieds d'emboitement dans l'adaptateur, respectivement, les premier et deuxième pieds d'emboitement présentant la même forme ;
- le kit comporte, en plus des premier et deuxième espaceurs, un troisième espaceur comportant une troisième ouverture orale présentant une aire sensiblement égale à l'aire de l'ouverture d'adaptateur, le troisième espaceur comportant de préférence un troisième pied d'emboitement présentant la même forme que les premier et deuxième pieds d'emboitement.

**Selon un neuvième aspect principal, particulièrement avantageux pour améliorer la précision des données acquises :**
- la représentation de l'espaceur sur la vue observée par l'objectif dans une configuration d'acquisition du téléphone portable, représente moins de 50%, de préférence moins de 40%, de préférence moins de 30%, de préférence moins de 20%, de préférence moins de 10%, de préférence moins de 5%, de la surface de ladite vue ;
- la configuration d'acquisition est la configuration par défaut du téléphone portable ou une configuration prédéterminée que l'utilisateur sélectionne ou une configuration déterminée par le téléphone portable spécifiquement pour l'utilisation du kit d'acquisition ;
- la configuration déterminée par le téléphone portable pour l'utilisation du kit d'acquisition est déterminée en fonction de l'espaceur ;
- la chambre est de forme générale tubulaire et rectiligne ;
- la chambre comporte un tronçon qui diverge vers l'ouverture orale, ledit tronçon divergent commençant à moins de 5 cm, de préférence à moins de 3 cm, de préférence à moins de 1 cm de l'ouverture d'adaptateur, commençant de préférence à l'ouverture d'adaptateur ou à l'ouverture d'acquisition ;
- ledit tronçon divergent s'étend sur plus de 3 cm, de préférence plus 4 cm, de préférence plus 4 cm et/ou moins de 10 cm le long de la chambre ;
- la chambre comporte un tronçon qui converge vers l'ouverture orale, ledit tronçon convergeant se terminant à moins de 5 cm, de préférence moins de 3 cm, de préférence moins de 2 cm, de préférence moins de 1 cm de l'ouverture orale, ledit tronçon convergeant s'étendant de préférence jusqu'à l'ouverture orale ;
- la longueur de la chambre, mesurée le long de l'axe de l'ouverture d'adaptateur est supérieure à 3 cm, de préférence supérieure à 5 cm, et/ou inférieure à 25 cm, de préférence inférieure à 15 cm.

**Selon un dixième aspect principal, particulièrement avantageux pour améliorer l'hygiène :**
- l'adaptateur est fixé de manière amovible sur l'espaceur et la distance entre l'ouverture d'acquisition et l'objectif est inférieure à 3 cm, de préférence inférieure à 2 cm, de préférence inférieure à 1 cm, de préférence inférieure à 0,5 cm ;
- l'espaceur comporte une extrémité logée, de préférence emboîtée dans un logement de forme complémentaire défini par l'adaptateur, ladite extrémité définissant une chemise de protection dudit logement ;
- la paroi de la chemise est continue, c'est-à-dire qu'elle ne comporte pas de trou traversant.

**Selon un onzième aspect principal, particulièrement avantageux pour améliorer les conditions d'acquisition :**
- l'ouverture orale s'étendant partiellement hors du plan passant en son centre et perpendiculaire à l'axe reliant le centre de l'ouverture orale et le centre de l'ouverture d'acquisition ;
- le rebord distal présente une extrémité latérale écartée dudit plan d'une distance supérieure à 5 mm et inférieure à 12 mm ;
- les deux extrémités latérales du rebord distal sont écartées dudit plan de la même distance ;
- l'ouverture orale présente une courbure autour d'un axe vertical dans la position de service, le rayon de ladite courbure étant supérieur à 20 mm, de préférence supérieur à 30 mm, de préférence supérieur à 40 mm, de préférence supérieur à 45 mm, de préférence supérieur à 47 mm, et/ou inférieur à 80 mm, de préférence inférieur à 70 mm, de préférence inférieur à 60 mm, de préférence inférieur à 58 mm, de préférence inférieur à 55 mm ;
- l'ouverture orale présente une courbure exclusivement autour dudit axe vertical ;
- le rapport entre la distance entre les extrémités latérales du rebord distal et ladite distance entre lesdites extrémités latérales et ledit plan est compris entre 5 et 8 ;
- le rebord distal s'étend dans le prolongement de l'ouverture orale ;
- dans un tronçon de l'espaceur s'étendant sur plus de 70%, plus de 80%, plus de 90%, plus de 95%, plus de 99%, de préférence 100% de la longueur de l'espaceur, l'espaceur présente une surface extérieure dépourvue d'arête saillante et/ou dépourvue d'arête rentrante, la longueur de l'espaceur étant mesurée le long d'un axe joignant les centres de l'ouverture d'acquisition et de l'ouverture orale, un tronçon de l'espaceur étant une fraction de l'espaceur délimitée par deux plans perpendiculaires audit axe ;
- dans ledit tronçon, l'espaceur présente une surface extérieure dépourvue d'arête saillante marquant une rupture de pente supérieure à 90° et/ou est dépourvue d'arête rentrante marquant une rupture de pente supérieure à 90° ;
- l'espaceur comportant
   - un corps de forme générale tubulaire définissant l'ouverture orale et
   - un rebord distal s'étendant vers l'extérieur à partir du corps et conformé pour être introduit entre les lèvres et les dents de l'utilisateur ;
- la surface extérieure de l'espaceur présente, en tout point de la zone de transition entre le rebord distal et le corps 40, un rayon de courbure supérieur à 1 mm, de préférence supérieur à 1,5 mm, de préférence supérieur à 2 mm, de préférence supérieur à 3 mm et/ou inférieur 4 cm ;
- le rebord distal est interrompu, ou présente une hauteur inférieure à 5 mm, de préférence inférieure à 4 mm, de préférence inférieure à 3 mm, de préférence inférieure à 2 mm, de préférence inférieure à 1 mm, de manière continue, sur une longueur supérieure à 3 mm, supérieure à 5 mm, voire supérieure à 10 mm, voire supérieure à 15 mm, et/ou inférieure à 50 mm, de préférence inférieure à 30 mm, de préférence inférieure à 20 mm ;
- ladite interruption ou ladite réduction de hauteur est centrée sur un frein labial dans la position de service, lorsque l'utilisateur maintient le kit d'acquisition devant lui, par exemple pour acquérir des images de ses incisives ;
- le rebord distal borde l'ouverture orale, c'est-à-dire s'étend à la périphérie de l'ouverture orale ;
- le rebord distal est plat ;
- le rebord distal s'étend radialement par rapport à l'axe de la chambre joignant les centres de l'ouverture d'adaptateur et de l'ouverture orale ;
- le rebord distal s'étend dans le prolongement de l'ouverture orale ;
- le rebord distal présente une hauteur maximale supérieure à 1 mm, supérieure à 2 mm, supérieure à 3 mm, et/ou inférieure à 10 mm, 8 mm, ou 6 mm ;
- la chambre comporte un tronçon qui diverge vers l'ouverture orale, ledit tronçon divergent commençant de préférence à moins de 5 cm de l'ouverture d'adaptateur et se terminant de préférence à l'ouverture orale ;
- ledit tronçon divergent est défini par une paroi présentant une épaisseur constante.

Bien entendu, les caractéristiques, optionnelles ou non, qui définissent les aspects principaux ci-dessus sont de préférence combinées entre elles.

L'invention concerne également un procédé d'acquisition de données 2D ou 3D au moyen d'un kit d'acquisition selon l'invention, ledit procédé comportant les étapes suivantes :
a) optionnellement, choix, par l'utilisateur, d'un espaceur dans un ensemble d'espaceurs disponibles, de préférence en fonction de marques de personnalisation portées par lesdits espaceurs disponibles et/ou de la morphologie de l'utilisateur ;
b) assemblage, de préférence par l'utilisateur, du kit d'acquisition, en particulier en fixant le téléphone portable sur l'adaptateur, et optionnellement l'espaceur sur l'adaptateur ;
c) introduction, par l'utilisateur, de l'extrémité de l'espaceur définissant l'ouverture orale dans la bouche de l'utilisateur, de préférence en introduisant le rebord de l'espaceur entre ses lèvres et ses dents ;
d) positionnement, par l'utilisateur, de l'ouverture orale en face de dents pour lesquelles lesdites données doivent être acquises ;
e) activation, de préférence par l'utilisateur, du téléphone portable de manière à acquérir lesdites données 2D ou 3D ;
f) optionnellement, modification du positionnement de l'ouverture orale, par exemple par rotation autour de l'utilisateur, puis reprise à l'étape e) ;
e) optionnellement, démontage du kit d'acquisition, de préférence par l'utilisateur ;
f) optionnellement, lavage et/ou désinfection de l'espaceur, voire seulement de l'espaceur.

De préférence, à l'étape d), l'espaceur est positionné de manière à ne pas prendre appui sur les gencives, ni sur les dents, ni sur un éventuel appareil orthodontique porté par les dents. De préférence, il n'est en contact qu'avec les lèvres et la face intérieure des joues.

Le rebord distal peut en particulier être à plus de 1 mm, à plus de 2 mm, à plus de 3 mm, et/ou à moins de 10 mm, de préférence à moins de 8 mm des dents.

### Définitions

Par « utilisateur », on entend toute personne pour laquelle un kit selon l'invention peut être mis en œuvre, que cette personne soit malade ou non, ou que cette personne soit en cours de traitement ou non. Un kit selon l'invention peut être utilisé pour un autre animal qu'un être humain.

Par « professionnel des soins dentaires », on entend un dentiste, un orthodontiste ou un laboratoire d'orthodontie.

On appelle "téléphone portable" ou « téléphone mobile » un appareil de type iPhone^{®}. Un tel appareil pèse typiquement moins de 500 g, est doté d'un appareil photo comportant un objectif lui permettant de prendre des films ou des photos, voire d'un scanner lui permettant d'acquérir des modèles numériques tridimensionnels. Un téléphone portable est en outre capable d'échanger des données avec un autre appareil éloigné de plus de 500 km du téléphone portable, et est capable d'afficher lesdites données.

La « configuration par défaut » d'un téléphone portable est constituée par l'ensemble des réglages de ce téléphone portable tels que définis avant sa première utilisation.

Par "image", on entend une image en deux dimensions, comme une photographie. Une image est formée de pixels.

Un « film » est considéré comme un ensemble de photos.

Par « modèle », on entend un modèle tridimensionnel numérique. Un modèle est constitué d'un ensemble de voxels.

Le téléphone portable permet d'acquérir des données « extraorales », c'est-à-dire sans que l'objectif du téléphone portable ne soit introduit dans la bouche de l'utilisateur. Les « données 2D » acquises par un téléphone portable sont des images, et en particulier classiquement des photos et des films. Les « données 3D » acquises par un téléphone portable sont des données qui fournissent des coordonnées dans les trois dimensions de l'espace. Les « données 3D » sont de préférence des modèles numériques tridimensionnels.

Un « écarteur » (« retractor » en anglais), ou « écarteur dentaire », est un dispositif destiné à retrousser les lèvres.

Il comporte un rebord supérieur et un rebord inférieur, et/ou un rebord droit et un rebord gauche, s'étendant autour d'une ouverture d'écarteur et destinés à être introduits entre les dents et les lèvres. En position de service, les lèvres de l'utilisateur sont en appui sur ces rebords, de sorte que les dents sont visibles à travers l'ouverture d'écarteur. Un écarteur permet ainsi d'observer les dents sans être gêné par les lèvres.

Les dents ne reposent cependant pas sur l'écarteur, de sorte que l'utilisateur peut, en tournant la tête par rapport à l'écarteur, modifier les dents qui sont visibles à travers l'ouverture d'écarteur. Il peut aussi modifier l'écartement entre ses arcades dentaires. En particulier, un écarteur n'appuie pas sur les dents de manière à écarter les deux mâchoires l'une de l'autre.

Dans un mode de réalisation, un écarteur est configuré de manière à écarter élastiquement l'une de l'autre les lèvres supérieure et inférieure de manière à dégager les dents visibles à travers l'ouverture d'écarteur.

Dans un mode de réalisation, un écarteur est configuré de manière que la distance entre le rebord supérieur et le rebord inférieur, et/ou entre le rebord droit et le rebord gauche soit constante.

Des écarteurs sont par exemple décrits dans PCT/EP2015/074896, US 6,923,761, ou US 2004/0209225.

« Fixer » une pièce sur une autre signifie établir un lien physique entre ces deux pièces, de sorte que tout déplacement de l'une de ces pièces finit par entrainer celui de l'autre pièce. Une pièce montée en translation, à rotation ou à rotule sur une autre pièce est « fixée » à cette autre pièce. Une fixation peut être « définitive » ou « de manière amovible », selon que les deux pièces sont adaptées ou non à une désolidarisation, de préférence à la main, par un utilisateur. Une fixation peut être rigide ou non, selon qu'elle autorise ou non un déplacement relatif des deux pièces fixées l'une à l'autre. On considère qu'un élément venu de matière avec un autre est « fixé » sur cet autre élément.

Deux pièces sont « mobiles élastiquement » l'une par rapport à l'autre, si leur position relative peut être modifiée sous l'action d'une force et si, lorsque cette force cesse, elles retrouvent leurs positions relatives initiales.

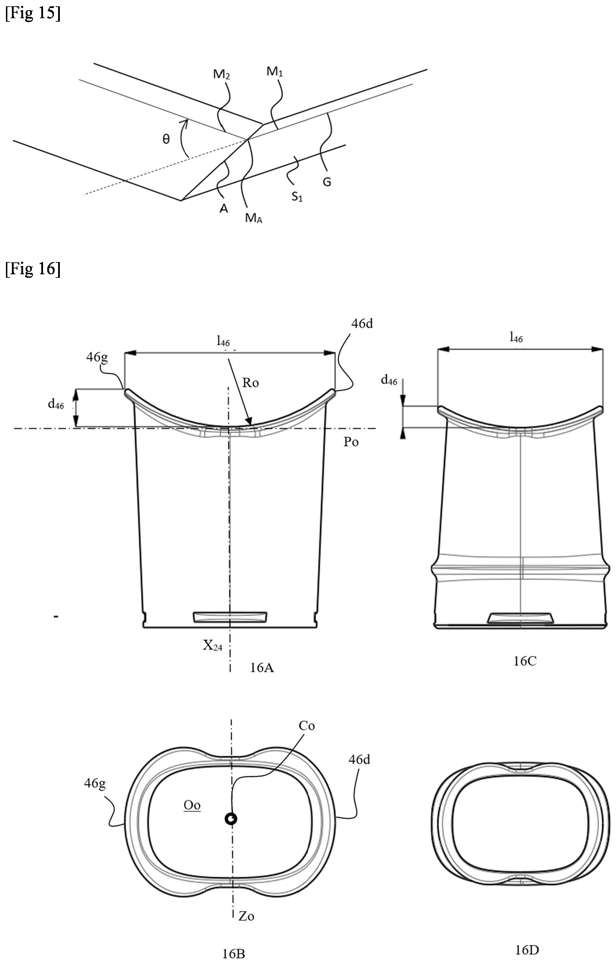

Une « arête » d'une surface est une ligne sur cette surface qui présente une longueur supérieure à 1 cm et qui est constituée de points auxquels une ligne de plus grande pente présente une évolution de pente, aussi appelée « rupture de pente », supérieure à 45°. Par extension, on dit également que l'angle de l'arête est supérieur à 45° ou que la surface présente une rupture de pente supérieure à 45°. Une arête est rentrante lorsqu'elle définit un renfoncement. Par exemple, la figure 15 représente une surface S₁ sur laquelle la ligne de plus grande pente G présente une rupture de pente de θ en tout point M_{A} de la ligne A. Si l'angle θ est supérieur à 45° pour tous les points de la ligne A, la ligne A est une arête. En pratique, on peut mesurer l'évolution de pente entre deux points M₁ et M₂ de la ligne de plus grande pente séparés d'une distance de 3 mm, la distance de 3 mm étant mesurée en suivant ladite ligne.

Une « position assemblée » est une position dans laquelle le téléphone portable est fixé rigidement sur l'adaptateur du kit, et l'espaceur est fixé rigidement sur l'adaptateur.

Une « position d'acquisition » est une position assemblée dans laquelle l'objectif du téléphone portable a une vue au moins partielle, de préférence complète, de l'ouverture orale, à travers l'ouverture d'adaptateur et l'ouverture d'acquisition.

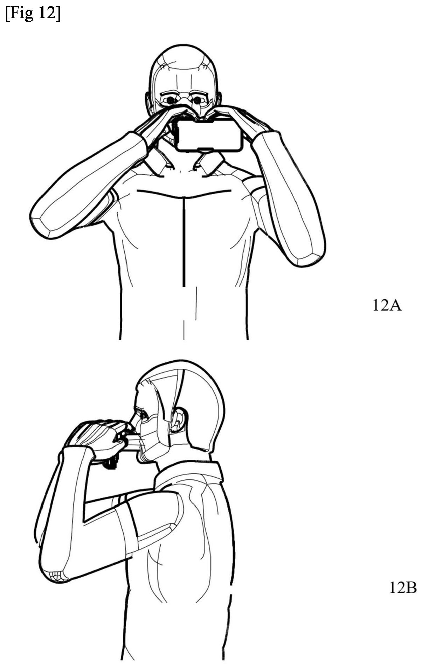

La « position de service » est la position dans laquelle l'espaceur du kit, en position d'acquisition, est partiellement introduit dans la bouche de l'utilisateur, comme illustré sur la figure 12.

La position « bouche fermée » est la position d'occlusion dans laquelle les dentures supérieure et inférieure de l'utilisateur sont en contact.

La position « bouche ouverte » est la position d'ouverture totale de la bouche.

Sauf indications contraires, les qualificatifs utilisés pour définir des positions ou des orientations dans l'espace, comme « horizontal », « vertical », « bas », « haut », « supérieur », « inférieur », « droite » ou « gauche », sont définies, à des fins de clarté, en référence à une position de service observée par un utilisateur, ayant la tête droite et ayant disposé ses lèvres autour de l'ouverture orale de l'espaceur.

Par exemple, un axe vertical est un axe qui, dans la position de service, est vertical.

Les extrémités latérales (droite et gauche) du rebord distal sont les points du rebord distal qui sont les plus à droite et à gauche de l'utilisateur dans la position de service.

Sauf indications contraires, les qualificatifs « extérieur » et « intérieur » font référence à des volumes ou à des contours fermés, pour désigner un élément hors du volume, par exemple hors d'une chambre, ou hors du contour fermé. Par exemple, la surface extérieure d'un corps tubulaire est la surface qui est exposée vers l'extérieur du corps tubulaire.

Les adjectifs "premier" et "deuxième" sont utilisés à des fins de clarté uniquement.

Par souci de clarté, un axe ou une direction qui, dans la position de service, s'étend horizontalement et perpendiculairement au plan général de l'ouverture orale, est référencé Xᵢ, « i » étant un index faisant référence à une pièce concernée par cet axe ou cette direction. Un axe ou une direction qui, dans la position de service, s'étend verticalement est référencé Zᵢ, « i » étant un index faisant référence à une pièce concernée par cet axe ou cette direction. Un axe ou une direction qui, dans la position de service, s'étend perpendiculairement aux axes Xᵢ et Zᵢ est référencé Yᵢ, « i » étant un index faisant référence à une pièce concernée par cet axe ou cette direction. Pour l'utilisateur, des axes Xᵢ, Yᵢ et Zᵢ sont donc des axes avant-arrière, droite-gauche, et verticaux, respectivement.

L'axe d'une ouverture est l'axe qui passe en son centre, perpendiculairement au plan général de l'ouverture.

Des première et deuxième droites sont dites perpendiculaires l'une à l'autre lorsque des premier et deuxième plans perpendiculaires aux dites première et deuxième droites, respectivement, sont perpendiculaires l'un à l'autre.

"Comprendre", "comporter" et "présenter" doivent être interprétés de manière large et non limitative, sauf indication contraire.

### Brève description des dessins

D'autres caractéristiques et avantages de l'invention apparaîtront encore à la lecture de la description détaillée qui va suivre et à l'examen du dessin annexé dans lequel :
[Fig 1] la figure 1 représente un kit selon l'invention, en perspective, en position assemblée ;
[Fig 2] la figure 2 représente l'espaceur du kit de la figure 1, en perspective ;
[Fig 3] la figure 3 illustre, pour le kit de la figure 1, le mouvement d'écartement du premier mors, depuis la position rapprochée (figure 3A) jusqu'à la position écartée (figure 3B), en passant par une position intermédiaire (figure 3C) ;
[Fig 4] la figure 4 représente l'adaptateur du kit de la figure 1 partiellement démonté de manière à illustrer le déplacement du premier mors, dans une position intermédiaire du premier mors, vu de face (figure 4A), de côté (figure 4B) et suivant la section BB, représentée sur la figure 4B (figure 4C) ;
[Fig 5] la figure 5 représente l'adaptateur partiellement démonté de la figure 4, dans la position rapprochée, vu de face (figure 5A), de côté (figure 5B) et en section BB, représentée sur la figure 5B (figure 5C) ;
[Fig 6] la figure 6 représente l'adaptateur du kit de la figure 1 partiellement démonté de manière à illustrer le déplacement de la platine sur l'embase, vu de face, sans téléphone portable (figure 6A), et avec le téléphone portable (figure 6B), l'ouverture d'adaptateur étant en position basse ;
[Fig 7] la figure 7 représente l'adaptateur partiellement démonté de la figure 6 vu de face, sans téléphone portable (figure 7A) et avec le téléphone portable (figure 7B), l'ouverture d'adaptateur étant en position haute, face à l'objectif du téléphone portable sur la figure 7B (position d'acquisition) ;
[Fig 8] la figure 8 représente une vue éclatée du support du kit de la figure 1 ;
[Fig 9] la figure 9 représente schématiquement un kit selon l'invention vu de côté ;
[Fig 10] la figure 10 représente schématiquement un kit selon l'invention vu de côté ;
[Fig 11] la figure 11 représente schématiquement un détail d'un espaceur d'un kit selon l'invention vu de côté ;
[Fig 12] la figure 12 illustre schématiquement la position de service, l'utilisateur étant vu de face (figure 12A) et de côté (figure 12B) ;
[Fig 13] la figure 13 représente une vue observée par l'objectif du téléphone portable ;
[Fig 14] la figure 14 représente en perspective l'adaptateur du kit de la figure 1 ;
[Fig 15] la figure 15 illustre la définition d'une arête ;
[Fig 16] la figure 16 représente un premier espaceur vu de dessus (figure 16A) et de face (figure 16B), et un deuxième espaceur, d'un même kit d'acquisition, vu de dessus (figure 16C) et de face (figure 16D).

Dans les différentes figures, les organes identiques ou analogues ont été désignés avec des références identiques.

Les figures sont fournies à des fins illustratives. Elles ne sont pas limitatives.

### Description détaillée

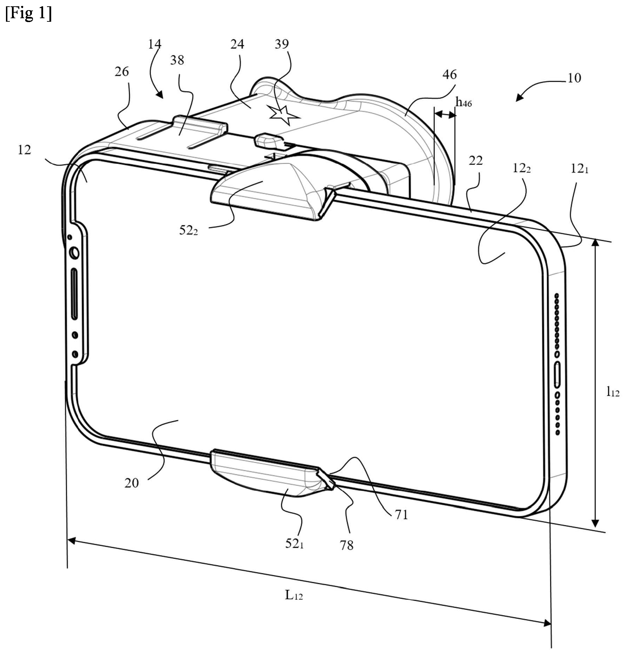

Le kit 10 représenté sur la figure 1 comporte un téléphone portable 12 et un support 14.

### Téléphone portable

Tout téléphone portable conventionnel peut être utilisé.

Le téléphone portable 12 est un appareil personnel, conventionnellement de forme sensiblement parallélépipédique, comportant classiquement un appareil photo.

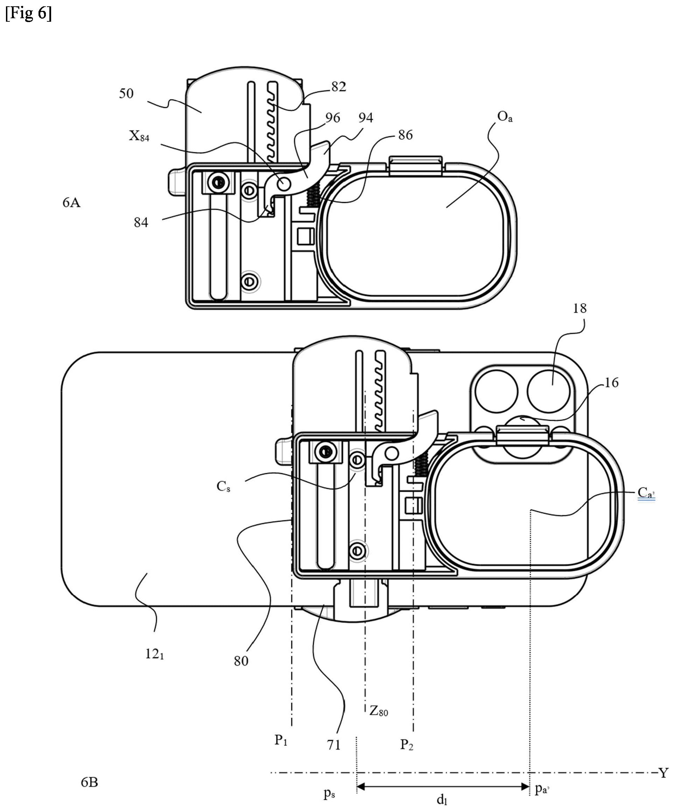

Un téléphone portable présente classiquement :
- une face avant 12₁ munie d'un objectif 16 et, de préférence un flash 18, comme illustré sur la figure 6,
- une face arrière 12₂ présentant un écran 20, et
- un chant 22, définissant l'épaisseur du téléphone et joignant les faces avant et arrière.

Les faces avant et arrière sont classiquement de forme générale rectangulaire, parallèles et de mêmes dimensions. Leur longueur L₁₂ est typiquement comprise entre 8 et 20 cm. Leur largeur l₁₂ est typiquement comprise entre 4 et 10 cm, comme illustré sur la figure 1.

L'épaisseur du téléphone portable est typiquement comprise entre 4 mm et 15 mm.

L'écran 20 recouvre sensiblement toute la face arrière. Il sert d'interface pour l'utilisateur. En particulier il lui permet de visualiser ce que « voit » l'objectif 16, c'est-à-dire la « vue » de l'objectif. De préférence, l'écran 20 est tactile et permet de commander des fonctions du téléphone portable.

L'objectif 16 présente classiquement un axe optique X₁₆ perpendiculaire à la face avant, et permet l'acquisition de données 2D, voire 3D. En particulier, il permet l'acquisition de photos en couleurs, et/ou de photos infrarouges. Les photos infrarouges permettent avantageusement de faire apparaître les dents avec un excellent contraste.

L'objectif 16 présente classiquement un diamètre supérieur à 1 mm et inférieur à 15 mm.

Il est parfois au centre du téléphone portable. Il est généralement disposé à proximité d'un côté de la face avant, par exemple à moins de 3 cm d'un petit côté de la face avant, classiquement à mi-longueur de ce côté, ou dans un coin de la face avant, par exemple à moins de 1 cm d'un angle de la face avant.

Le téléphone portable 12 peut comporter, sur la face avant, plusieurs objectifs, par exemple 2, plus de 2, plus de 3 ou plus de 4 objectifs 16 et/ou moins de 8, ou moins de 6 objectifs.

Le flash 18 peut être à proximité de l'objectif 16, de préférence à moins de 4 cm, moins de 3 cm, moins de 2 cm, ou moins de 1 cm et/ou à plus de 1 mm de l'objectif 16. Le téléphone portable 12 peut comporter, sur la face avant, plusieurs flashs, par exemple plus de 2, plus de 3 ou plus de 4 flashs et/ou moins de 8, ou moins de 6 flashs.

Dans un mode de réalisation préféré, le téléphone portable comporte un programme informatique permettant de guider l'utilisateur lors des opérations, par exemple pour lui indiquer comment assembler ou utiliser le kit, par exemple pour lui indiquer la position et/ou l'orientation à donner au support pour prendre des photos particulières.

Le téléphone portable 12 est fixé, de préférence de manière amovible, sur le support 14.

### Support

Le support 14 a une double fonction. Il permet simultanément
- d'écarter les lèvres de l'utilisateur pour en dégager les dents, et
- de faciliter le positionnement et l'orientation du téléphone portable par rapport aux dents.

Il comporte un espaceur 24 et un adaptateur 26.

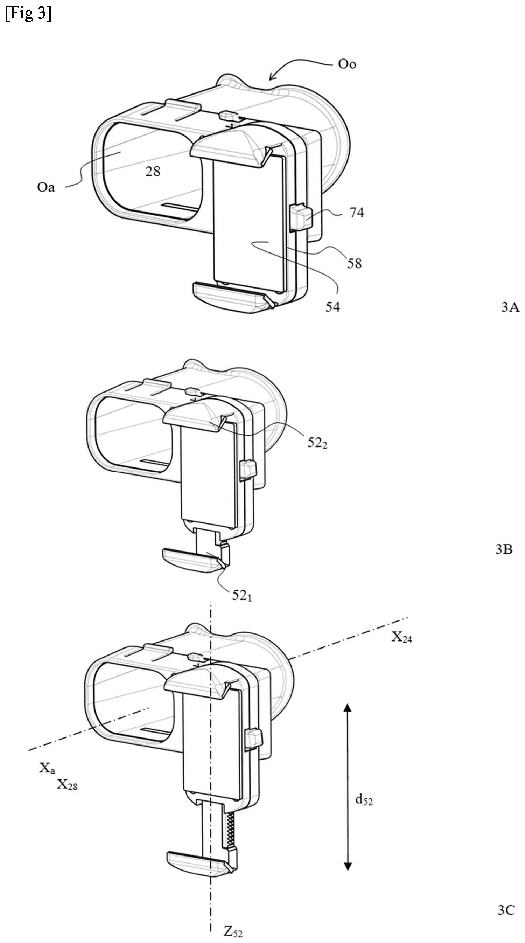

Dans la position assemblée, l'espaceur 24 et l'adaptateur 26 définissent ensemble une chambre 28 (voir figure 3) débouchant, de préférence exclusivement, par une ouverture orale Oo et par une ouverture d'adaptateur Oa, en regard de l'objectif 16 du téléphone portable 12.

La chambre 28 est de préférence sensiblement tubulaire, de préférence rectiligne d'axe X₂₈.

La longueur de la chambre 28, mesurée le long de l'axe Xa de l'ouverture d'adaptateur, est de préférence supérieure à 3 cm, de préférence supérieure à 5 cm, et/ou inférieure à 25 cm, de préférence inférieure à 20 cm, de préférence inférieure à 15 cm, de préférence inférieure à 10 cm, de préférence inférieure à 8 cm, de préférence inférieure à 7 cm.

De préférence, la chambre 28 ne débouche vers l'extérieur que par les ouvertures orale Oo et d'adaptateur Oa, et, de préférence encore, le téléphone portable 12 obture l'ouverture d'adaptateur Oa. Avantageusement, la luminosité de l'environnement extérieur n'influence pas l'acquisition des données (images ou modèles 3D) par le téléphone portable.

De préférence, le support ne comporte aucun moyen d'éclairage artificiel, l'éclairage étant de préférence exclusivement fourni par le flash du téléphone portable 12.

L'ouverture orale définie par l'espaceur 24 peut s'étendre dans un plan vertical Po, passant en son centre Co, de préférence perpendiculairement à l'axe X₂₄ joignant les centres des ouvertures orale Oo et d'acquisition Oa'.

De préférence cependant, comme illustré sur la figure 16, l'ouverture orale s'étend partiellement hors du plan Po, du côté opposé au corps 40 de l'espaceur, c'est-à-dire du côté de l'utilisateur dans la position de service.

De préférence, l'ouverture orale Oa est incurvée autour d'un axe Zo vertical dans la position de service, de préférence exclusivement autour d'un axe vertical Zo, de manière à se rapprocher des dents dans la position de service.

Le rebord distal 46, de préférence l'espaceur 24, est(sont) de préférence symétrique(s) par rapport à un plan vertical qui, dans la position de service, se confond avec le plan sagittal. L'axe Zo est de préférence inclus dans ce plan vertical et perpendiculaire à l'axe X₂₄.

De préférence, le rayon de courbure autour de l'axe vertical Zo est supérieur à 20 mm, de préférence supérieur à 30 mm, de préférence supérieur à 40 mm, de préférence supérieur à 45 mm, et/ou inférieur à 80 mm, de préférence inférieur à 70 mm, de préférence inférieur à 60 mm, de préférence inférieur à 55 mm, de préférence d'environ 50 mm.

Un rayon de courbure entre 47 mm et 58 mm est particulièrement approprié.

La courbure de l'ouverture orale ne permet pas au rebord distal 46 de suivre la denture, mais offre plusieurs avantages dans pour l'acquisition extraorale de données 2D ou 3D.

Des essais ont montré notamment qu'une telle courbure limite le risque que les lèvres ne passent par-dessus le rebord distal et viennent obturer l'ouverture orale lorsque le kit est orienté, à droite ou à gauche de l'utilisateur, pour l'acquisition de données 2D ou 3D, et en particulier de photographies, face aux molaires ou aux prémolaires (positions d'observation latérales). Dans ces positions à droite ou à gauche de l'utilisateur, cette courbure évite également que l'espaceur n'entre en butée, par exemple avec une attache d'un appareil orthodontique. Cette courbure évite aussi que l'espaceur ne vienne pincer les joues lorsque l'ouverture orale est replacée face aux incisives.

Les essais ont également encore montré que cette courbure limite la traction sur les lèvres dans les positions d'observation latérales, ce qui améliore le confort, et donc l'observance du traitement orthodontique éventuel.

Par « incurvé », on entend une forme générale permettant de suivre un arc, c'est-à-dire de suivre la forme d'au moins une partie d'un « C ». De préférence, la courbure est continue, sans rupture de pente, de manière à ne pas créer des arêtes rentrantes.

L'ouverture d'adaptateur Oa peut être définie par l'adaptateur 26 et/ou par l'espaceur 24.

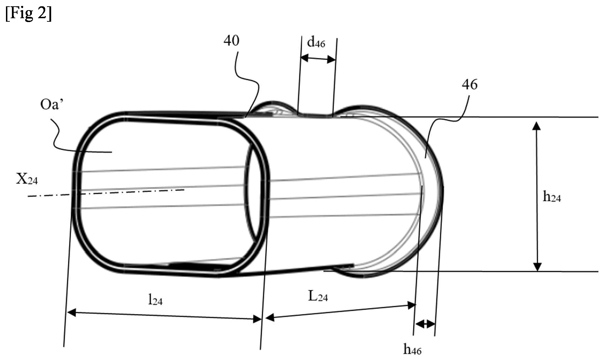

De préférence, l'ouverture d'adaptateur Oa est définie par l'adaptateur 26 et l'espaceur 24 définit une ouverture d'acquisition Oa' (voir figure 2).

Dans un mode de réalisation préféré, l'espaceur 24 présente la forme d'un corps tubulaire débouchant exclusivement par les ouvertures orale Oo et d'acquisition Oa'.

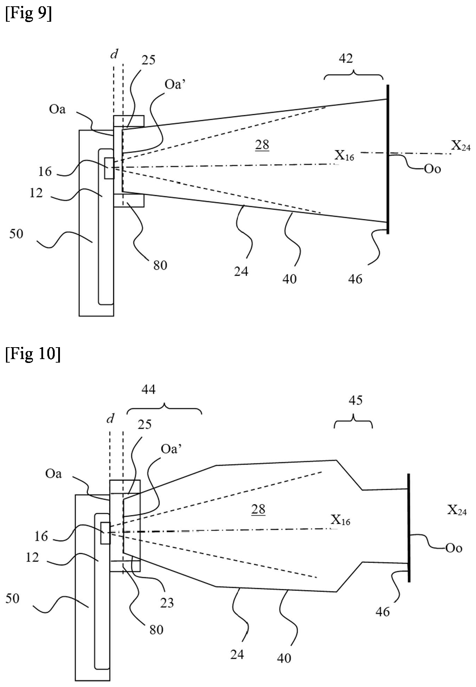

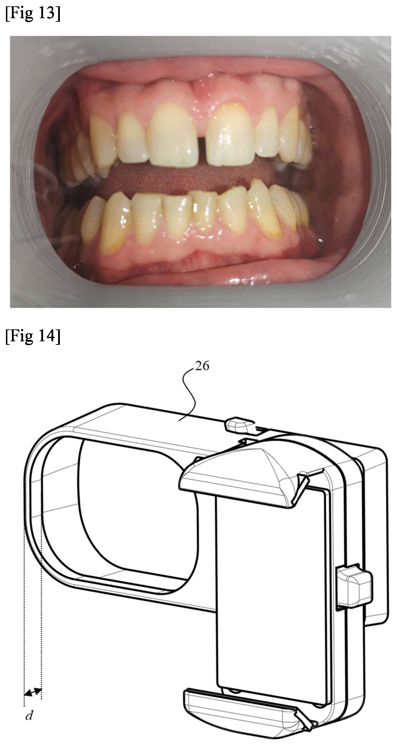

De préférence, comme schématiquement représenté sur la figure 9, dans la position assemblée, la distance d entre l'ouverture d'acquisition Oa' de l'espaceur 24 et l'ouverture d'adaptateur Oa est inférieure à 3 cm, de préférence inférieure à 2 cm, de préférence inférieure à 1 cm, de préférence inférieure à 0,5 cm, de préférence inférieure à 0,2 cm, de préférence sensiblement nulle. La distance d est également représentée sur la figure 14. De préférence, l'ouverture d'acquisition Oa' de l'espaceur et l'ouverture d'adaptateur Oa sont coplanaires. Avantageusement, l'espaceur 24 forme une chemise qui protège l'adaptateur de l'air rejeté par l'utilisateur, et en particulier des postillons. L'hygiène en est améliorée.

De préférence, l'ouverture d'acquisition Oa' de l'espaceur et l'ouverture d'adaptateur Oa sont sensiblement de mêmes dimensions.

De préférence, comme illustré sur la figure 10, une extrémité 23 de l'espaceur 24 est logée, de préférence emboîtée dans un logement 25, de préférence de forme complémentaire. Le logement 25 définit l'ouverture d'adaptateur Oa. De préférence, l'espaceur 26 traverse ce logement sur toute sa longueur.

Au moins ladite extrémité de l'espaceur 24, de préférence tout l'espaceur 24 est défini par une paroi pleine, c'est-à-dire que l'espaceur ne débouche vers l'extérieur que par l'ouverture orale et l'ouverture d'acquisition.

Dans un mode de réalisation, l'adaptateur 26 et l'espaceur 24 forment un ensemble monobloc, l'adaptateur 26 étant rigidement fixé sur l'espaceur 24 de manière définitive, voire étant venu de matière avec l'espaceur.

L'adaptateur 26 est de préférence rigidement fixé sur l'espaceur 24 de manière amovible.

Avantageusement, l'adaptateur 26 peut être utilisé pour plusieurs espaceurs.

Avantageusement également, l'espaceur 26 peut être nettoyé ou stérilisé indépendamment de l'adaptateur.

En particulier dans ce mode de réalisation, l'espaceur 24 est de préférence en un matériau autoclavable, de préférence à l'autoclave ou au lave-vaisselle. De préférence, l'espaceur peut être stérilisé plus de 50 fois sans se dégrader. On considère que la stérilité est atteinte lorsque la probabilité d'isoler un germe est inférieure à 1 pour 1 million, suivant le niveau d'assurance de stérilité (NAS) réglementé par la norme EN 556.

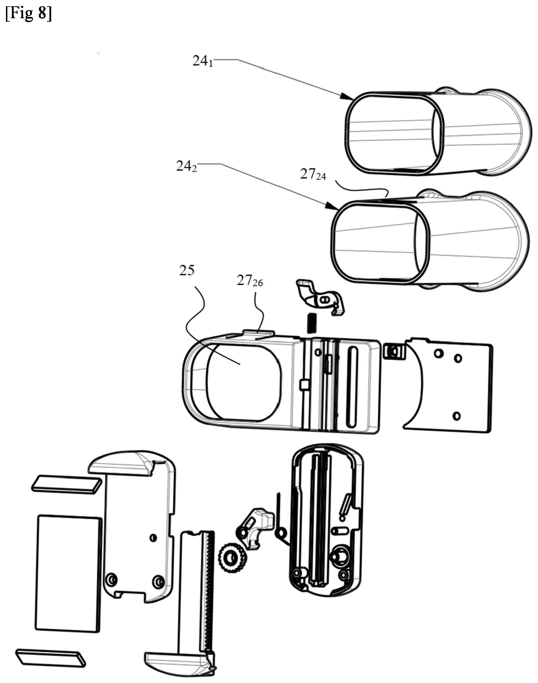

De préférence, l'adaptateur 26 est rigidement fixé sur l'espaceur 24 au moyen d'une attache qui comporte des parties d'attache d'adaptateur 27₂₆ et d'espaceur 27₂₄, comme illustré sur la figure 8. Les parties d'attache d'adaptateur et d'espaceur coopèrent entre elles pour assurer une fixation désactivable de l'adaptateur sur l'espaceur. Elles peuvent donc être accouplées ou désaccouplées pour fixer ou détacher l'adaptateur de l'espaceur, respectivement.

De préférence, la partie d'attache d'espaceur 27₂₄ est immobile sur l'espaceur 24, c'est-à-dire qu'elle n'est pas prévue pour être déplacée lors de l'assemblage et le désassemblage avec la partie d'attache d'adaptateur. De préférence, la partie d'attache d'espaceur 27₂₄ n'est pas déformée lors de l'assemblage et le désassemblage avec la partie d'attache d'adaptateur. Elle conserve sa forme lorsqu'elle est accouplée et/ou désaccouplée de la partie d'attache d'adaptateur 27₂₆. Par exemple, elle peut être formée dans la paroi de l'espaceur 24 qui délimite la chambre 28, par un bourrelet de matière ou par un renfoncement non traversant. On considère que la partie d'attache d'espaceur conserve sa forme lorsqu'aucun point de sa surface ne se déplace, lors de l'assemblage et du désassemblage, de plus de 1 mm. De préférence, aucun point de la surface de la partie d'attache d'espaceur ne se déplace, lors de l'assemblage et du désassemblage, de plus de 0,5 mm, de préférence de plus de 0,2 mm.

L'immobilité de la partie d'attache d'espaceur 27₂₄, que l'on peut qualifier de « passive », permet avantageusement d'éviter sa déformation lors d'une opération de stérilisation de l'espaceur 24. Cette caractéristique est particulièrement avantageuse lorsque l'adaptateur 26 n'a pas besoin d'être stérilisé, par exemple parce qu'il est protégé par l'espaceur 24.

De préférence, la partie d'attache d'adaptateur 27₂₆ est mobile par rapport au reste de l'adaptateur. Par exemple, elle peut être formée par une griffe, adaptée pour crocheter un bourrelet de matière de l'espaceur ou pour s'insérer élastiquement dans un renfoncement de l'espaceur 24, comme visible sur la figure 8.

La partie d'attache d'adaptateur 27₂₆ peut être également une partie de l'adaptateur déformable élastiquement, par exemple un bourrelet de matière déformable élastiquement. Dans un mode de réalisation, l'adaptateur porte un ou plusieurs bourrelets, par exemple quatre bourrelets, disposés de manière à s'insérer élastiquement dans un ou plusieurs renfoncements respectifs ménagés à la surface extérieure du corps de l'espaceur lors de l'assemblage.

De préférence, la partie d'attache d'espaceur 27₂₄ et la partie d'attache d'adaptateur 27₂₆ sont en un matériau rigide, c'est-à-dire non déformable lorsqu'on appuie dessus à la main, à la différence d'une matière élastique comme un élastomère. De préférence, ce matériau présente une dureté supérieure à 70 ShoreD et/ou inférieure à 100 ShoreD.

De préférence, le kit, de préférence au moins l'espaceur 24, porte une pièce de personnalisation portant la marque de personnalisation 39 fixée, de préférence de manière amovible, sur l'adaptateur 26 et/ou sur l'espaceur 24.

La marque de personnalisation 39 comporte de préférence un identifiant de l'utilisateur et/ou d'un cabinet d'un professionnel de soins dentaires et/ou d'une société, par exemple un groupement de professionnels de soins dentaires.

La marque de personnalisation 39 peut également comporter une information relative à une pathologie de l'utilisateur.

La pièce de personnalisation peut être fixée par tout moyen connu, notamment au moyen d'un ou plusieurs clip(s), vis, bande(s) auto agrippante(s) de type Velcro^{®}, bande(s) élastique(s), aimant(s), crochet(s), verrou(s), sangle(s), ventouse(s), ou d'un matériau adhésif.

La marque de personnalisation peut être également est inscrite, ou gravée, sur l'espaceur ou l'adaptateur, de préférence sur l'espaceur.

L'inscription peut être par exemple réalisée au moyen d'un crayon, par exemple marqueur indélébile, en particulier sur un espaceur en carton, ou par tampographie.

L'identifiant peut être par exemple constitué d'une couleur, de caractères alphanumériques, d'un motif géométrique ou non ou d'une microstructure particulière.

La marque de personnalisation 39 peut comporter une représentation esthétique, par exemple un bijou, ou ludique, par exemple la représentation d'un personnage de bande dessinée.

De préférence, la marque de personnalisation 39 est en une couleur ou en un matériau différent de la couleur ou du matériau, respectivement, de l'espaceur 24 et/ou l'adaptateur 26 qui la porte(nt).

La marque de personnalisation 39 peut par exemple représenter plus de 0,1%, plus de 0,5%, plus de 1%, plus de 3 % et/ou moins de 90%, moins de 50%, moins de 20%, moins de 10% de la surface de l'espaceur 24 et/ou de l'adaptateur 26 qui la porte(nt).

Dans un mode de réalisation, le kit comporte plusieurs espaceurs amovibles, référencés 24₁ et 24₂ sur la figure 8, assemblables à l'adaptateur 26 et présentant des ouvertures orales et/ou des pièces de personnalisation différentes. Avantageusement, l'adaptateur 26 peut être utilisé pour plusieurs personnes différentes, chaque personne utilisant un espaceur 24 qui lui est propre.

Avantageusement, l'utilisateur s'approprie plus facilement le kit, ce qui l'incite à l'utiliser. L'observance d'un traitement nécessitant l'utilisation d'un kit d'acquisition selon l'invention en est améliorée.

En outre, l'adaptateur peut être utilisé pour plusieurs espaceurs, par exemple affecté chacun à un enfant d'une fratrie.

### Espaceur

L'espaceur 24 a pour fonction de définir un espacement entre le téléphone portable fixé sur l'adaptateur 26 et l'ouverture orale Oo ainsi qu'une orientation du téléphone portable par rapport à l'ouverture orale. Avantageusement, dans la position de service, les données (images ou modèles 3D) acquises par le téléphone portable 12 à travers l'objectif 16 sont ainsi acquises à une distance prédéterminée des dents de l'utilisateur et selon une orientation prédéfinie. De préférence, l'espaceur 24 est configuré pour que cet espacement et cette orientation soit constants.

L'espaceur 24 comporte un corps 40, débouchant à ses extrémités par les ouvertures orale Oo et d'acquisition Oa'.

Le corps 40 présente de préférence la forme générale d'un tube, de section constante ou variable, de préférence sensiblement rectiligne d'axe X₂₄, débouchant à ses extrémités par les centres des ouvertures orale Oo et d'acquisition Oa'. L'espaceur 24 peut en particulier présenter une forme générale d'un tube cylindrique, par exemple de section circulaire, ovale ou polygonale, par exemple rectangulaire, ou d'un tube tronconique.

La section transversale de l'espaceur 24, c'est-à-dire dans un plan de coupe perpendiculaire à l'axe X₂₄, présente de préférence une forme générale sensiblement identique tout au long du tube, par exemple rectangulaire ou ovale ou ronde.

L'espaceur 24 est de préférence conformé de manière qu'en l'absence de toute utilisation d'un zoom du téléphone portable 12, la représentation de l'espaceur 24 sur la vue observée par l'objectif 16 (illustrée, sur les figures 9 et 10, par les deux droites en traits interrompus divergents depuis l'objectif 16), représente moins de 50%, de préférence moins de 40%, de préférence moins de 30%, de préférence moins de 20%, de préférence moins de 10%, de préférence moins de 5% de la surface de ladite vue. Autrement dit, sur une image acquise par le téléphone portable 12, la représentation de l'espaceur 24 occupe une surface limitée.

La figure 13 donne un exemple d'une telle vue. La représentation de l'espaceur est la zone grise.

Avantageusement, la précision de la zone utile représentant les dents en est améliorée.

De préférence, la représentation de l'espaceur 24 sur ladite vue représente une partie 42 de l'espaceur qui s'étend exclusivement à moins de 5 cm, 3 cm, 2 cm, 1 cm de l'ouverture orale Oo, comme illustré sur la figure 9.

Avantageusement, la mise au point sur les dents en est améliorée.

Pour limiter la surface occupée par la représentation de l'espaceur 24 sur la vue observée par l'objectif 16, il est possible de limiter la longueur de l'espaceur 24. L'espaceur 24 doit cependant avoir une taille minimale pour qu'une mise au point soit possible.

La « configuration d'acquisition » est constituée par l'ensemble des réglages du téléphone portable qui déterminent l'acquisition des données 2D ou 3D par l'objectif 16 du téléphone portable. Dans un mode de réalisation, la configuration d'acquisition est la configuration par défaut du téléphone portable 12.

Pour limiter la surface occupée par la représentation de l'espaceur 24 sur la vue observée par l'objectif 16, alternativement ou en complément à une limitation de la longueur de l'espaceur 24, il est possible de configurer le téléphone portable selon une configuration d'acquisition spécifiquement adaptée à l'utilisation du kit d'acquisition.

La configuration d'acquisition est de préférence définie par le téléphone portable lui-même, c'est-à-dire automatiquement.

De préférence, la configuration d'acquisition est obtenue par un réglage du zoom du téléphone portable. De préférence, le zoom est réglé par le téléphone portable, sans intervention de l'utilisateur à cet effet. Par exemple, le zoom peut être réglé lorsqu'un programme chargé dans le téléphone portable est lancé pour l'utilisation du kit d'acquisition.

De préférence, la configuration d'acquisition, de préférence le zoom, est réglé automatiquement en fonction de l'espaceur, par exemple en fonction de sa forme, ou de sa longueur, ou de sa largeur. Dans un mode de réalisation, l'espaceur est reconnu par le téléphone portable, par exemple par lecture d'une marque disposée sur l'espaceur ou par une analyse de la vue observée par l'objectif faisant apparaître une partie de l'espaceur, dans la position d'acquisition.

Dans un mode de réalisation, la configuration d'acquisition, de préférence le zoom, est définie automatiquement en fonction d'une distance mesurée, dans la position de service, par exemple pour réaliser la mise au point.

Pour limiter la surface occupée par la représentation de l'espaceur 24 sur la vue observée par l'objectif 16, alternativement ou en complément à une limitation de la longueur de l'espaceur 24 et à un réglage de la configuration d'acquisition, et en particulier du zoom du téléphone portable, il est encore possible d'agir sur la forme de la chambre 28, et en particulier sur la forme de l'espaceur 24.

La chambre 28, et de préférence au moins l'espaceur 24, comporte(nt) de préférence au moins un tronçon 44 divergeant, de préférence de manière régulière, vers l'ouverture orale Oa. Dans un mode de réalisation, le tronçon divergent 44 commence à moins de 5 cm, moins de 3 cm, moins de 1 cm de l'ouverture d'acquisition Oa', de préférence à moins de 5 cm, moins de 3 cm, moins de 1 cm de l'ouverture d'adaptateur Oa.

De préférence, le tronçon divergent 44 définit l'ouverture d'acquisition Oa', de préférence l'ouverture d'adaptateur Oa.

De préférence, la divergence est suffisante pour que, dans la configuration d'acquisition, par exemple dans la configuration par défaut du téléphone portable 12, le tronçon divergent ne soit pas visible par l'objectif 16 dans la position d'acquisition.

Pour limiter l'encombrement, la chambre 28, de préférence l'espaceur 24 comporte de préférence au moins un tronçon 45 convergeant, de préférence de manière régulière, vers l'ouverture orale Oa. Dans un mode de réalisation, le tronçon convergent se termine à moins de 5 cm, moins de 3 cm, moins de 1 cm de l'ouverture orale, de préférence définit l'ouverture orale.

Dans un mode de réalisation, l'espaceur 24 comporte un dit tronçon divergent 44 et un dit tronçon convergent 45. Avantageusement, la représentation de l'espaceur 24 sur la vue observée par l'objectif 16 peut être réduite, ce qui facilite la mise au point par le téléphone portable 12.

De préférence, au moins dans la zone de l'espaceur 24 qui est visible sur la vue observée par l'objectif 16, l'espaceur 24 présente une couleur grise.

De préférence, la couleur grise comporte, en code RGB (Red, Green, Blue) sensiblement la même valeur pour le rouge, le vert et le bleu, l'écart maximal entre les valeurs pour le rouge, le vert et le bleu étant de préférence inférieur à 10, de préférence inférieur à 5, de préférence sensiblement nul. De préférence, ces valeurs sont comprises entre 50 et 210, de préférence entre 70 et 190, de préférence encore entre 90 et 170, de préférence entre 140 et 160. Par exemple, la couleur grise peut être proche de (120, 120, 120), ou (150, 150,150).

De manière surprenante, les inventeurs ont découvert que l'analyse de la couleur des dents sur des photos en couleurs réalistes en est facilitée et améliorée. Les photos acquises sont donc bien adaptées à une analyse colorimétrique.

Sans être liés par cette théorie, les inventeurs expliquent ce résultat du fait que la couleur grise est une couleur neutre, qui n'altère pas les couleurs lors d'une acquisition. En effet, les réglages classiquement utilisés par les téléphones portables peuvent modifier les couleurs de la photo, notamment en rééquilibrant la balance des blancs.

Comme illustré sur la figure 2, la longueur L₂₄ de l'espaceur 24, mesurée le long de l'axe X₂₄, est de préférence supérieure à 3 cm, de préférence supérieure à 4 cm, de préférence supérieure à 5 cm, et/ou inférieure à 25 cm, de préférence inférieure à 20 cm, de préférence inférieure à 15 cm, de préférence inférieure à 10 cm, de préférence inférieure à 8 cm, de préférence inférieure à 7 cm.

La largeur l₂₄ du corps 40 de l'espaceur 24, c'est-à-dire se plus grande dimension dans un plan transversal, en considérant l'ensemble des plans transversaux le long de l'axe X₂₄, est de préférence supérieure à 2 cm et/ou inférieure à 10 cm.

La hauteur h₂₄ du corps 40 de l'espaceur 24, c'est-à-dire se plus petite dimension dans un plan transversal, en considérant l'ensemble des plans transversaux le long de l'axe X₂₄, est de préférence supérieure à 2 cm, de préférence supérieure à 4 cm, de préférence supérieure à 6 cm, de préférence supérieure à 7 cm et/ou inférieure à 10 cm, de préférence inférieure à 9 cm.

L'espaceur 24 comporte de préférence un rebord distal 46 s'étendant vers l'extérieur, à la périphérie de l'ouverture orale Oa et à partir de la surface extérieure du corps 40, apte à être introduit entre les lèvres et les dents de l'utilisateur.

Avantageusement, les lèvres peuvent être ainsi écartées pour ne pas obturer l'ouverture orale Oa. En outre, le rebord distal 46 facilite la rétention de l'espaceur 24 dans la bouche de l'utilisateur.

Le rebord distal 46 est de préférence venu de matière avec le corps 40 de l'espaceur 24, ce qui limite les coûts de fabrication et limite le nombre d'arêtes. L'hygiène en est améliorée.

Le rebord distal 46 peut être surmoulé sur le corps 40.

Il peut être en un élastomère, pour un confort optimal.

De préférence, le rebord distal 46 est plat, c'est-à-dire présente la forme d'une lame, de préférence d'épaisseur constante.

De préférence, le rebord distal 46 s'étend sensiblement radialement par rapport à l'axe X₂₄ de l'ouverture orale Oa, c'est-à-dire dans un plan perpendiculaire à l'axe X₂₄. Avantageusement, l'acquisition de données avec le téléphone portable 12 est ainsi possible, de manière ergonomique, alors que l'espaceur 24 est maintenu horizontalement.

De préférence, comme illustré sur la figure 16, le rebord distal s'étend dans le prolongement de l'ouverture orale. Lorsque l'ouverture orale est incurvée, il peut être ainsi incliné par rapport à un plan perpendiculaire à l'axe X₂₄.

Le rebord distal 46 peut ceinturer totalement ou partiellement le corps 40.

De préférence, il comporte une portion droite et une portion gauche, destinés à prendre appui sur les commissures droite et gauche des lèvres de l'utilisateur, et qui définissent des extrémités latérales droite 46d et gauche 46g, respectivement.

La distance l₄₆ entre les extrémités latérales droite 46d et gauche 46g est de préférence supérieure à 40 mm, de préférence supérieure à 50 mm, et/ou de préférence inférieure à 80 mm, de préférence inférieure à 70 mm. Avantageusement, ces dimensions permettent, dans la position de service, de déplacer latéralement l'espaceur à droite et à gauche de l'utilisateur, mais aussi permettent à l'utilisateur de rapprocher ses mâchoires jusqu'à la position d'occlusion dans laquelle les dentures supérieure et inférieure de l'utilisateur sont en contact.

De préférence, l'ouverture orale est incurvée de manière que des extrémités latérales droite 46d et gauche 46g soient écartées d'une distance d₄₆, de préférence d'une même distance d₄₆, du plan Po, comme illustré sur la figure 16. La distance d₄₆ est de préférence supérieure à 5 mm et inférieure à 12 mm.

La corde qui relie les extrémités latérales droite 46d et gauche 46g est de préférence parallèle au plan Po.

De préférence, le rapport l₄₆/d₄₆ entre la distance entre les extrémités latérales du rebord distal et la distance entre lesdites extrémités latérales et le plan Po est compris entre 5 et 8. Quand le kit d'acquisition comporte plusieurs espaceurs différents, lesdits espaceurs présentent de préférence desdits rapports différents.

Le rebord distal 46 peut être interrompu, comme dans le mode de réalisation représenté, ou comporter une encoche, de manière à laisser libre le frein labial présent au niveau des arcades supérieure et inférieure de l'utilisateur. L'interruption ou l'encoche permettent en outre le déplacement de l'espaceur 24 dans la bouche de l'utilisateur, notamment des rotations autour des arcades dentaires pour l'acquisition de photos ou de modèles 3D en vues latérales.

L'encoche peut par exemple présenter une forme triangulaire, arrondie ou rectangulaire.

La longueur d₄₆ de l'interruption ou de l'encoche est de préférence supérieure à 3 mm, supérieure à 5 mm, voire supérieure à 10 mm, voire supérieure à 15 mm, et/ou inférieure à 50 mm, de préférence inférieure à 30 mm, de préférence inférieure à 20 mm (figure 2).

La hauteur maximale h₄₆ du rebord distal 46 est de préférence supérieure à 1 mm, supérieure à 2 mm, supérieure à 3 mm, et/ou inférieure à 10 mm, 8 mm, ou 6 mm.

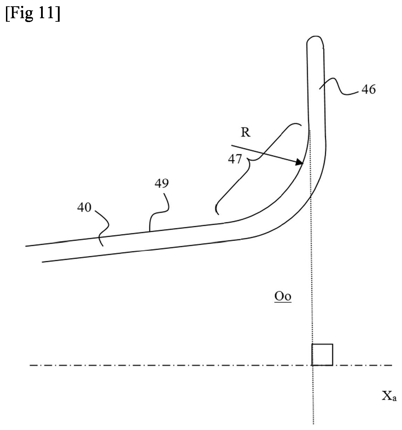

Comme illustré sur la figure 11, dans la zone de transition 47 entre le rebord distal 46 et le corps 40, la surface extérieure 49 de l'espaceur 24 présente de préférence, en tout point, un rayon de courbure R supérieur à 1 mm, supérieur à 1,5 mm, supérieur à 2 mm, de préférence supérieur à 3 mm, de préférence supérieur à 5 mm, de préférence supérieur à 7 mm, de préférence supérieur à 9 mm, de préférence supérieur à 10 mm, de préférence supérieur à 20 mm, de préférence supérieur à 30 mm, et/ou inférieur à 4 cm (figure 11). Avantageusement, la zone de transition ne définit donc pas d'arête rentrante, ce qui facilite le nettoyage et améliore encore les conditions d'hygiène.

De préférence, comme représenté sur la figure 11, l'épaisseur de la paroi de l'espaceur est constante au moins dans la partie qui porte le rebord distal 46, de préférence sur toute la longueur de l'espaceur. En particulier, la chambre 28 comporte un tronçon 44 qui diverge vers l'ouverture orale Oa, ledit tronçon divergent commençant à moins de 5 cm de l'ouverture d'adaptateur Oa et se terminant à l'ouverture d'adaptateur Oa.

Le tronçon divergent est de préférence défini par une partie de la paroi de l'espaceur qui présente une épaisseur constante.

Dans un mode de réalisation, le rebord distal 46 est amovible. En particulier, il peut être réalisé au moyen d'un écarteur dentaire.

De préférence, le corps 40, de préférence tout l'espaceur 24 est en un matériau opaque. Avantageusement, l'environnement extérieur ne perturbe pas l'éclairage des dents par le téléphone portable 12. Le corps 40 protège également l'intimité de l'utilisateur.

Le corps 40 peut être en un matériau rigide, par exemple en polyéthylène PE, en polytéréphtalate d'éthylène PET, en polystyrène PS ou en polypropylène PP ou souple, par exemple en carton.

Dans un mode de réalisation, le corps 40, de préférence tout l'espaceur 24 est en un matériau recyclable, de préférence biodégradable, de préférence selon la norme européenne EN13432, avec un résidu après 6 mois inférieur à 10%, de préférence inférieur à 5%, de préférence inférieur à 1%. De préférence, plus de 50%, 70%, 80%, ou 90% de l'espaceur 24, en pourcentage en poids, est biodégradable.

Le matériau peut être en particulier un matériau fibreux, de préférence cellulosique, de préférence du carton.

L'espaceur peut être à usage unique, ou être jetable.

De préférence encore, l'espaceur 24 est monobloc. Avantageusement, son coût de fabrication en est limité.

### Adaptateur

L'adaptateur 26, représenté notamment sur la figure 14, a pour fonction de fixer, de manière amovible, le téléphone portable au support 14, et de le positionner dans une position d'acquisition permettant à l'objectif 16 du téléphone portable 12 d'observer l'ouverture orale Oa à travers l'ouverture d'adaptateur Oa et l'ouverture d'acquisition Oa' de l'espaceur. De préférence, l'adaptateur 26 est configuré pour que cette position soit modifiable.

Le téléphone portable 12 peut être fixé sur l'adaptateur 26 par exemple avec une ou plusieurs bandes élastiques, une ou plusieurs bandes auto agrippantes, un ou plusieurs clips, une ou plusieurs vis, un volet, par exemple monté à rotation sur l'embase de l'adaptateur et verrouillable dans une position dans laquelle il serre le téléphone portable 12, des mors, une ou plusieurs ventouses ou un ou plusieurs aimants.

Le volet peut être maintenu contre le téléphone portable pour le maintenir en position, par tout moyen, par exemple au moyen d'un ou plusieurs clip(s), vis, bande(s) auto agrippante(s) de type Velcro^{®}, bande(s) élastique(s), aimant(s), crochet(s), verrou(s), sangle(s), ventouse(s), en particulier microventouse(s) ou nanoventouse(s), ou d'un matériau adhésif, par exemple un gel.

Le téléphone portable peut être également introduit dans une coque, une housse ou un coffret, fixé sur l'embase de l'adaptateur. Le coffret par exemple en plastique, peut être muni d'une porte montée à rotation, de préférence autour d'un axe horizontal ou vertical. La forme de la housse ou du coffret n'est pas limitative, pourvu qu'elle permette à l'objectif 16 d'observer l'extérieur de la housse ou du coffret et, de préférence, de laisser visible l'écran depuis l'extérieur de la housse ou du coffret.

Le coffret ou la housse ou la coque peuvent être par exemple fixés sur l'embase par tout moyen, par exemple par complémentarité de forme, ou au moyen d'un ou plusieurs clip(s), vis, bande(s) auto agrippante(s) de type Velcro^{®}, bande(s) élastique(s), aimant(s), crochet(s), verrou(s), sangle(s), ventouse(s), en particulier microventouse(s) ou nanoventouse(s), ou d'un matériau adhésif, par exemple un gel. Le coffret ou la housse peuvent être venus de matière avec l'embase.

Dans un mode de réalisation, l'adaptateur comporte une sangle, de préférence élastique, de préférence en un élastomère, de préférence transparente, pour fixer le téléphone portable.

Cette liste d'exemples n'est pas limitative.

Une surface de l'écran 20 « dégagée » est une surface qui n'est pas recouverte par l'adaptateur 26. Si une partie de l'adaptateur 26 en un matériau transparent, par exemple une sangle transparente, recouvre une surface de l'écran, cette surface n'est pas considérée comme « dégagée ».

L'adaptateur 26 est de préférence configuré pour, dans la position assemblée, laisser dégagée plus de 80%, de préférence plus de 90%, de préférence plus de 95%, de préférence sensiblement 100% de la surface de l'écran 20 du téléphone portable.

On appelle « surface de contact » la surface de l'adaptateur 26 qui est en contact avec le téléphone portable 12 dans la position assemblée.

De préférence, plus de 70%, plus de 80%, de préférence plus de 90%, de préférence sensiblement 100% de la surface de contact est en contact sur la face avant 12₁ et le chant 22 du téléphone portable, de préférence sur le chant 22 du téléphone portable.

Une portion de la surface de contact peut être en contact avec la face arrière du téléphone portable, voire avec l'écran 20. De préférence, l'aire de cette portion de la surface de contact représente moins de 20%, de préférence moins de 10%, de préférence moins de 5%, de préférence moins de 2% de l'aire de ladite face arrière, de préférence est nulle. De préférence, l'aire de cette portion de la surface de contact représente moins de 20%, de préférence moins de 10%, de préférence moins de 5%, de préférence moins de 2% de l'aire dudit écran 20.

De préférence, la surface de contact ne traverse pas l'écran 20 depuis un bord de la face arrière 12₂ jusqu'à un bord opposé de la face arrière 12₂. En particulier, la surface de contact ne traverse pas l'écran verticalement. De préférence, dans la position assemblée, le téléphone portable n'est pas ceinturé par l'adaptateur 26, comme c'est le cas lorsqu'une sangle ou un volet prennent appui sur la face arrière pour comprimer le téléphone portable contre une face de réception de l'adaptateur 26.

Une surface de l'écran 20 « visible » est une surface qui n'est pas recouverte par l'adaptateur 26 ou qui est recouverte par une partie transparente de l'adaptateur, de manière qu'il soit possible de voir l'écran à travers. Si une partie de l'adaptateur 26 en un matériau transparent, par exemple une sangle transparente, recouvre une surface de l'écran, cette surface est donc considérée comme « visible ». De préférence, une communication tactile avec l'écran est possible à travers lesdites parties de l'adaptateur 26 en un matériau transparent.

Une surface de l'écran 20 qui n'est pas « visible » est dite « masquée ».

L'adaptateur 26 est de préférence configuré pour, dans la position assemblée, laisser visible plus de 80%, de préférence plus de 90%, de préférence plus de 95%, de préférence sensiblement 100% de la surface de l'écran 20 du téléphone portable.

Avantageusement, l'écran 20 permet ainsi d'observer parfaitement les dents. En outre, toute l'interface homme-machine tactile est avantageusement accessible.

De préférence encore, l'adaptateur 26 est configuré pour autoriser la fixation de différents téléphones portables, et en particulier de téléphones portables présentant des dimensions différentes.

Dans un mode de réalisation préféré, le téléphone portable 12 est maintenu sur l'adaptateur 26 par serrage.

En particulier, de préférence, l'adaptateur 26 comporte une embase 50, un premier mors 52₁ et un deuxième mors 52₂, opposé au premier mors, au moins le premier mors étant monté mobile sur l'embase 50 entre des positions rapprochée et écartée dans lesquelles le premier mors est à des distances minimale et maximale du deuxième mors, respectivement.

Dans la position écartée, la distance d₅₂ entre les premier et deuxième mors, maximale, est de préférence supérieure à 5 cm, de préférence supérieure à 7 cm, de préférence supérieure à 9 cm, et/ou inférieure à 25 cm, de préférence inférieure à 15 cm (figure 3).

Dans la position rapprochée, la distance d₅₂ entre les premier et deuxième mors, minimale, est de préférence supérieure à 2 cm, de préférence supérieure à 3 cm, de préférence supérieure à 5 cm, et/ou inférieure à 10 cm, de préférence inférieure à 8 cm.

Le rapprochement du premier mors du deuxième mors, ou « mouvement de rapprochement », conduit à la position assemblée, généralement intermédiaire entre les positions rapprochée et écartée, dans laquelle les premier et deuxième mors prennent en étau et serrent le téléphone portable 12, de préférence par appui sur le chant 22 du téléphone portable.

Le premier mors 52₁ est de préférence monté mobile en translation par rapport au deuxième mors, suivant une direction de serrage Z₅₂, de préférence verticale dans la position de service.

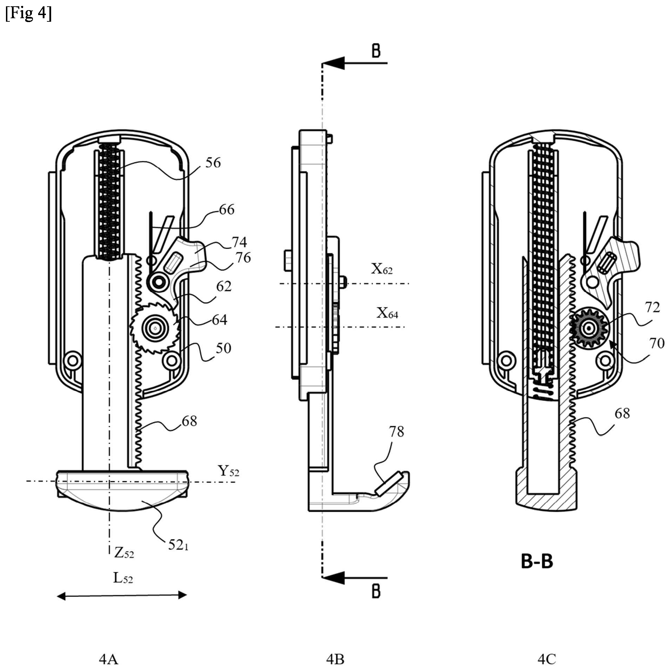

De préférence, le premier mors 52₁ est monté élastiquement mobile en translation par rapport au deuxième mors 52₂, suivant la direction de serrage. De préférence, l'adaptateur 26 comporte un organe déformable élastiquement, de préférence un ressort 56, de préférence un ressort de compression, disposé de manière à s'opposer à une poussée du premier mors 52₁ vers la position rapprochée. Sur la figure 4, le ressort 56 est disposé dans un logement de l'embase 50 et s'étendant verticalement. Il est comprimé entre le fond du logement et le premier mors 52₁. L'adaptateur 26 peut comporter plusieurs ressorts 56 disposés dans des logements respectifs s'étendant verticalement et comprimés entre le fond de leur logement respectif et le premier mors 52₁. Avantageusement, le risque de porte-à-faux en est réduit.

Le deuxième mors 52₂ peut être mobile sur l'embase 50. Cependant, de préférence, l'embase 50 intègre le deuxième mors, comme représenté sur la figure 3 (sur la figure 4, le deuxième mors a été démonté de l'embase 50).

Comme représenté sur la figure 4B, en vue de côté, au moins un des premier et deuxième mors, de préférence chacun des premier et deuxième mors présente la forme d'une gouttière, ou « forme de berceau », qui s'étend selon la direction Y₅₂ et qui, dans la position assemblée, reçoit le téléphone portable 12.

Avantageusement, les premier et deuxième mors viennent ainsi non seulement serrer le téléphone portable, mais aussi le retiennent par complémentarité de forme. Le maintien du téléphone portable 12 en est amélioré. La forme en gouttière favorise également un coulissement du téléphone portable entre les premier et deuxième mors, parallèlement à la direction Y₅₂. Un tel coulissement permet de disposer l'objectif 16 de manière que l'axe optique X₁₆ soit dans le plan vertical contenant l'axe Xₐ de l'ouverture d'adaptateur Oa.

De préférence, l'embase 50 comporte une face de réception 54, de préférence plane, de préférence verticale, sur laquelle le téléphone portable prend appui dans la position assemblée. Cette surface plane est de préférence définie par un patin 58 en élastomère. Elle s'étend de préférence jusqu'au deuxième mors. Avantageusement, l'appui du téléphone portable sur la face de réception 54 contribue à son maintien, notamment en limitant le glissement entre les premier et deuxième mors.

De préférence, la mobilité du téléphone portable 12 positionné entre les premier et deuxième mors n'est pas limitée par butée, ce qui permet de positionner un téléphone portable d'une longueur quelconque.

De préférence, au moins le premier mors 52₁ est monté incrémentalement mobile sur l'embase 50.

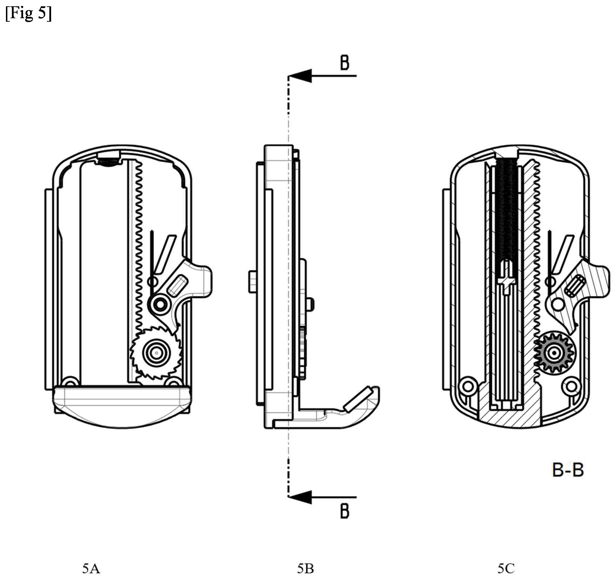

De préférence, l'adaptateur 26 comporte un premier système anti-retour désactivable empêchant un mouvement d'écartement du premier mors, c'est-à-dire un mouvement opposé à un mouvement de rapprochement. Le premier système anti-retour comporte de préférence, comme illustré sur les figures 4 et 5 :
- un premier cliquet 62 monté à rotation, autour d'un axe X₆₂, sur l'embase 50, de manière à être désactivable,
- une roue à rochet 64 monté à rotation, autour d'un axe X₆₄, sur l'embase 50, coopérant avec le premier cliquet 62, et mécaniquement couplée au premier mors 52₁ de manière qu'une rotation de la roue à rochet 64 entraine un déplacement du premier mors parallèlement à la direction de serrage Z₅₂, et
- un premier ressort 66 poussant élastiquement le premier cliquet 62 contre les dents de la roue à rochet 64 de manière à n'autoriser une rotation de la roue à rochet 64 que dans le sens de rotation produisant un mouvement de rapprochement.

Les positions successives du premier mors lors d'une translation selon la direction de serrage Z₅₂, dites « positions indexées », sont marquées par la pénétration élastique du premier cliquet 62 entre les dents successives de la roue à rochet 64.

Le couplage de la roue à rochet 64 et du premier mors 52₁ peut être assuré avec un ensemble pignon-crémaillère.

De préférence, le premier mors 52₁ est pourvu d'une première crémaillère 68 s'étendant parallèlement à la direction de serrage et l'adaptateur 26 comporte une roue étagée 70 montée à rotation, autour de l'axe X₆₄, sur l'embase 50, et comportant
- la roue à rochet 64 et
- un pignon 72, coaxial à la roue à rochet 64, coopérant avec la première crémaillère 68 de manière qu'une rotation du pignon 72 entraine un déplacement du premier mors parallèlement à la direction de serrage.

Avantageusement, une telle roue étagée est particulièrement compacte et permet un déplacement incrémental et bien contrôlé du premier mors.

L'adaptateur 26 comporte de préférence un premier bouton poussoir 74 sur lequel un appui provoque la désactivation du premier système anti-retour.

De préférence, le premier cliquet 62 est couplé au premier bouton poussoir 74 de manière que l'actionnement du bouton poussoir (par pression sur le bouton poussoir) provoque le dégagement du premier cliquet 62 à l'écart de la roue à rochet. Dans un mode de réalisation préféré représenté sur la figure 4, le premier bouton poussoir 74 et le premier cliquet 62 sont chacun à une extrémité d'une bascule 76 montée à rotation autour de l'axe X₆₂. L'appui sur le premier bouton poussoir 74 provoque donc une rotation de la bascule 76 et le dégagement du premier cliquet 62 hors des dents de la roue à rochet 64, c'est-à-dire une désactivation du premier système anti-retour. La cessation de cet appui provoque une rotation dans le sens opposé, sous l'action du premier ressort 66, et l'engagement du premier cliquet 62 entre les dents de la roue à rochet 64.

Le premier système anti-retour forme ainsi un verrou pour sélectivement bloquer en position ou libérer le mouvement du premier mors 52₁ sur l'embase 50.

Comme décrit précédemment, de préférence, le premier mors 52₁ est monté mobile sur l'embase 50 de manière à s'écarter élastiquement du deuxième mors. La désactivation du premier système anti-retour provoque donc un retour élastique vers une position écartée.

Avantageusement, un retour élastique du premier mors 52₁ vers la position écartée facilite considérablement le montage du téléphone portable 12 sur l'adaptateur 26.

De préférence, au moins un des premier et deuxième mors, de préférence chacun des premier et deuxième mors comporte un tampon 78 en un élastomère qui, dans la position assemblée, est en appui sur le téléphone portable 12.

De préférence, le tampon 78 est configuré de manière que, lorsque le premier mors est incrémentalement mobile sur l'embase 50 et que le tampon 78 commence à serrer le téléphone portable, sa déformation élastique permette d'atteindre la position indexée suivante. Avantageusement, le serrage du téléphone portable permet de serrer efficacement le téléphone, sans l'endommager. Le tampon 78 contribue également à la protection du téléphone portable contre les chocs et les vibrations.

De préférence, la zone de serrage 71 par laquelle le téléphone portable 12 est serrée par les premier et deuxième mors est exclusivement définie par des tampons 78.

La zone de serrage 71 est constituée par un ensemble de points de serrage. A chacun de ces points, l'adaptateur exerce une force de compression, dite « de serrage », sur le téléphone portable 12. La plus grande distance entre des points de serrage, mesurée suivant la direction de la longueur L₁₂ du téléphone portable, est de préférence supérieure à 1 cm, de préférence supérieure à 2 cm, supérieure à 3 cm, et/ou inférieure à 20 cm, de préférence inférieure à 10 cm, de préférence inférieure à 8 cm, de préférence inférieure à 6 cm, de préférence inférieure à 5 cm.

De préférence, au moins une zone de serrage « élémentaire » (c'est-à-dire la partie de la zone de serrage définie par un mors), de préférence chaque zone de serrage élémentaire, présente une longueur hors tout L₅₂ supérieure à 1 cm, de préférence supérieure à 2 cm, supérieure à 3 cm, et/ou inférieure à 20 cm, de préférence inférieure à 10 cm, de préférence inférieure à 8 cm, de préférence inférieure à 6 cm, de préférence inférieure à 5 cm. Par « longueur hors tout », on entend la plus grande distance entre deux points de la zone de serrage élémentaire, suivant la direction Y₅₂. En particulier, la longueur hors tout n'est pas modifiée si la zone de serrage élémentaire est localement interrompue entre ces deux points.

Dans le mode de réalisation illustré sur la figure 4, la longueur hors-tout L₅₂ est la plus grande distance entre des points de serrage.

Avantageusement, la fixation du téléphone portable 12 sur l'adaptateur 26 est particulièrement stable. Le kit peut ainsi être manipulé en tenant le téléphone, ce qui facilite l'acquisition de données à travers l'objectif 16 du téléphone portable.

La zone de serrage élémentaire du premier mors et/ou du deuxième mors s'étend de préférence suivant la longueur du téléphone portable.

De préférence, comme illustré sur la figure 6, l'adaptateur 26 comporte encore une platine 80 définissant l'ouverture d'adaptateur Oa et montée mobile sur l'embase 50, de préférence au moins en translation, de préférence au moins parallèlement à une direction de coulissement Z₈₀ verticale, de préférence exclusivement parallèlement à la direction de coulissement Z₈₀.

Le coulissement peut être réalisé au moyen d'un coulisseau, par exemple porté par la platine 80, monté coulissant dans un rail ménagé dans l'embase 50. Un coulissement au moyen de plusieurs coulisseaux montés coulissant dans des rails respectifs parallèles peut être réalisé pour limiter le risque de porte-à-faux.

Avantageusement, la position de l'ouverture d'adaptateur Oa par rapport à l'objectif 16 peut être ainsi modifiée.

De préférence, la mobilité de la platine 80 sur l'embase 50 est indépendante de la mobilité du premier mors sur l'embase 50, et plus généralement indépendante de la position du téléphone portable 12 sur l'adaptateur 26.

Le téléphone portable 12 est fixé rigidement sur la platine 80 par serrage entre les premier et deuxième mors, de manière amovible.

La position du téléphone portable 12 par rapport à l'ouverture d'adaptateur Oa peut donc être réglée indépendamment horizontalement par glissement entre les premier et deuxième mors, et verticalement, par coulissement de la platine 80 sur l'embase 50, jusqu'à positionner l'objectif 16 face à l'ouverture d'adaptateur Oa, de préférence sensiblement au centre de l'ouverture d'adaptateur Oa.

L'ouverture d'adaptateur Oa est de préférence dimensionnée de manière que, au moins dans cette position centrée de l'objectif 16, le flash 18 fasse face à l'ouverture d'adaptateur Oa. Avantageusement, le flash 18 peut ainsi éclairer les dents lors de l'acquisition de données 2D ou 3D par le téléphone portable 12.

La platine 80 est de préférence montée incrémentalement mobile sur l'embase 50. Autrement dit, au moins dans un sens, elle peut être déplacée par pas, de préférence par pas constant. Avantageusement, le réglage de la position de l'ouverture d'adaptateur Oa par rapport au téléphone portable en est facilité.

De préférence, l'adaptateur 26 comporte un verrou désactivable permettant de sélectivement bloquer en position la platine 80 par rapport à l'embase 50.

De préférence, l'embase 50 comporte une deuxième crémaillère 82 et la platine 80 comporte
- un deuxième cliquet 84 monté à rotation, autour d'un axe X₈₄, sur la platine 80, de manière à coopérer avec les dents de la deuxième crémaillère 82,
- un deuxième ressort 86 poussant élastiquement le deuxième cliquet 84 contre les dents de la deuxième crémaillère 82 de manière à définir des positions indexées pour positionner la platine 80 le long de la deuxième crémaillère 82. Ces positions successives indexées sont marquées par la pénétration élastique du deuxième cliquet 84 entre les dents successives de la deuxième crémaillère 82.

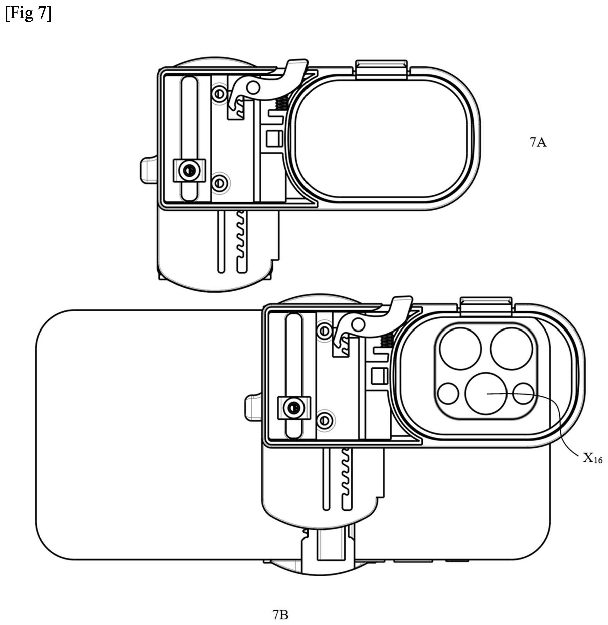

De préférence, la deuxième crémaillère 82 et le deuxième cliquet 84 sont conformés pour n'autoriser une translation de la platine 80 que dans un sens, à savoir, sur les figures 6 et 7, le sens de la montée.

La deuxième crémaillère 82 et le deuxième cliquet 84 définissent alors un deuxième système anti-retour désactivable empêchant un mouvement de retour, en l'occurrence un mouvement de descente de la platine 80 par rapport à l'embase 50.

L'adaptateur 26 comporte de préférence un deuxième bouton poussoir 94 sur lequel un appui provoque la désactivation du deuxième système anti-retour.

De préférence, le deuxième cliquet 84 est couplé au deuxième bouton poussoir 94 de manière que l'actionnement (par pression sur le deuxième bouton poussoir 94) provoque le dégagement du deuxième cliquet 84 hors de la deuxième crémaillère 82. Dans un mode de réalisation préféré représenté sur les figures 6 et 7, le deuxième bouton poussoir 94 et le deuxième cliquet 84 sont chacun à une extrémité d'une bascule 96 montée à rotation autour de l'axe X₈₄. L'appui sur le deuxième bouton poussoir 94 provoque donc une rotation de la bascule 96 et le dégagement du deuxième cliquet 84 hors des dents de la deuxième crémaillère 82, c'est-à-dire une désactivation du deuxième système anti-retour. La cessation de cet appui provoque une rotation dans le sens opposé, sous l'action du deuxième ressort 86, et l'engagement du deuxième cliquet 84 entre les dents de la deuxième crémaillère 82.

Le deuxième système anti-retour forme ainsi un verrou pour sélectivement bloquer en position ou libérer le mouvement de la platine 80 sur l'embase 50.

Bien entendu, de manière équivalente, la deuxième crémaillère 82 pourrait être sur la platine 80 et le deuxième cliquet 84 être monté sur l'embase 50.

Dans un mode de réalisation préféré, un repère, réalisé sur l'adaptateur, indique des positions possibles du téléphone portable 12 par rapport à l'embase 50 et/ou de la platine 80 par rapport à l'embase 50, par exemple en fonction du type ou de la marque du téléphone portable. Le repère peut être par exemple une flèche associée à une marque de téléphone portable.

De préférence, la platine 80 comporte un premier repère et l'embase comporte un ou plusieurs deuxièmes repères en face desquels le premier repère peut être positionné, ou, de manière équivalente, l'embase 50 comporte un premier repère et la platine 80 comporte un ou plusieurs deuxièmes repères en face desquels le premier repère peut être positionné. De préférence, les deuxièmes repères correspondent à des marques de téléphone portable. Autrement dit, pour positionner correctement un téléphone portable sur le support, il suffit de positionner le premier repère en face du deuxième repère correspondant à la marque de ce téléphone portable.

De préférence, l'ouverture d'acquisition Oa' est latéralement décalée par rapport à la zone de serrage du téléphone portable 12 sur l'adaptateur 26, comme représenté sur la figure 6B.

Le décalage latéral est déterminé en observant de face l'adaptateur 26, c'est-à-dire parallèlement à l'axe de l'ouverture d'acquisition Oa' (figure 6B). Sur la figure 6B, les plans verticaux P₁ et P₂ délimitent la zone de serrage.

La direction de décalage Y est la direction horizontale, parallèle au plan de la face avant du téléphone portable.

Le long de la direction de décalage Y, on peut identifier les positions p_{a'} et pₛ du centre Ca' de l'ouverture d'acquisition Oa' et du centre Cs de la zone de serrage, respectivement. La distance *dₗ*, selon la direction de décalage Y, mesure le décalage latéral (figure 6B).

De préférence, le décalage latéral est constant.

De préférence, le décalage latéral est supérieur à 1 cm, de préférence supérieur à 1 cm, de préférence supérieur à 3 cm, voire supérieur à 4 cm, ou supérieur à 5 cm, et/ou et inférieur à 10 cm.

Avantageusement, le décalage latéral permet à l'ouverture d'acquisition Oa' d'être positionnée face à l'objectif 16 et permet au téléphone portable d'être bien fixé sur l'adaptateur 26 même si l'objectif 16 est très proche d'un bord de la face avant. Cette fixation est notamment efficace car la zone de serrage peut être sensiblement à mi-longueur du téléphone portable 12 alors que l'ouverture d'acquisition ou l'ouverture d'adaptateur est proche du bord latéral du téléphone portable 12.

### Fonctionnement

Le fonctionnement du kit découle directement de la description qui précède.

Initialement, le kit est en position désassemblée. Le cas échéant, l'utilisateur choisit un espaceur 24 parmi plusieurs espaceurs, en fonction de la marque de personnalisation 39.

Il clipse l'espaceur 24 sur l'adaptateur 26, en introduisant l'extrémité de l'espaceur 24 opposée à l'ouverture orale Oa à l'intérieur du logement de forme complémentaire défini par l'adaptateur.

Le clipsage résulte de l'accrochage des griffes de l'adaptateur dans les renfoncements ménagés à la surface extérieure de l'espaceur 24.

Après clipsage, l'espaceur s'étend sensiblement jusqu'à l'ouverture d'adaptateur Oa définie par ce logement. Il définit ainsi une chemise qui limite le contact de la paroi définissant ce logement avec l'air respiré en service.

Si les premier et deuxième mors sont trop proches l'un de l'autre pour placer le téléphone portable, l'utilisateur appuie sur le premier bouton poussoir 74 pour dégager le premier cliquet 62 de la roue à rochet 64. Sous l'effet du ressort de compression 56, le premier mors 52₁ s'écarte du deuxième mors et rejoint la position écartée. Puis l'utilisateur cesse d'appuyer sur le premier bouton poussoir 74.

La distance d₅₂ entre les premier et deuxième mors est alors supérieure à la largeur du téléphone portable 12. L'utilisateur pose ensuite le téléphone portable 12 sur le patin 58, en appui sur le deuxième mors 52₂, puis le déplace latéralement, le long du deuxième mors, jusqu'à ce que l'ouverture d'adaptateur Oa soit sensiblement au droit de l'objectif 16, c'est-à-dire au-dessus ou en-dessous de l'objectif 16. Le décalage latéral des premier et deuxième mors par rapport à l'ouverture d'adaptateur permet d'atteindre cette position sans substantiellement décentrer le serrage par rapport au milieu de la longueur du téléphone portable.

Ensuite, l'utilisateur repousse le premier mors jusqu'à ce qu'il vienne en contact avec le chant du téléphone portable, de manière à le prendre en étau avec le deuxième mors.

Sous l'effet de ce rapprochement, la première crémaillère 68 entraine le pignon 72, et donc la roue étagée 70 et la roue à rochet 64. Le premier cliquet 62, poussé par le premier ressort 66, engrène la roue à rochet 64 et empêche tout mouvement d'écartement des premier et deuxième mors. Il permet aussi un déplacement incrémental du premier mors, c'est-à-dire « par pas ».

Lorsque le premier mors arrive en contact avec le chant du téléphone portable, la réaction du téléphone portable s'oppose à la poursuite mouvement de rapprochement. La déformation des tampons 78 permet cependant de pousser le premier mors au moins jusqu'au cran suivant (engrènement avec du premier cliquet 62 avec la dent suivante de la roue à rochet).

Le téléphone portable 12 est alors fixé rigidement sur l'embase 50, serré entre les premier et deuxième mors. L'écran 20 est bien dégagé.

L'utilisateur ajuste alors la position verticale de la platine 80 sur l'embase 50, de manière que l'ouverture d'adaptateur soit sensiblement en face de l'objectif 16.

Dans le mode de réalisation des figures 6 et 7, le deuxième cliquet 84, poussé par le deuxième ressort 86, engrène les dents de la deuxième crémaillère 82 et empêche tout mouvement de la platine 80 vers le bas. Il permet un déplacement incrémental de la platine 80.

Si la platine 80 est trop haute, l'utilisateur appuie sur le deuxième bouton poussoir 94 pour dégager le deuxième cliquet 84 de la deuxième crémaillère 82. Il peut alors faire descendre la platine 80. Dès qu'il relâche le deuxième bouton poussoir 94, le cliquet engrène à nouveau la deuxième crémaillère 82, sous l'effet du deuxième ressort 86.

Une fois que l'ouverture d'adaptateur Oa est sensiblement en face de l'objectif 16, et de préférence du flash, le kit, en position d'acquisition, est prêt à être utilisé.

L'utilisateur active alors le téléphone portable de manière qu'il puisse servir à l'acquisition d'images ou de modèles 3D. Il peut avantageusement se servir de l'écran 20, bien dégagé.

Il introduit ensuite le rebord distal 46 entre ses lèvres et ses dents et positionne l'ouverture orale en face des dents pour lesquelles il veut acquérir des données, de préférence prendre des photos ou acquérir un scan. Il est alors dans une position de service. En appuyant sur le déclencheur du téléphone portable, l'utilisateur acquiert ces données.

Il déplace ensuite l'ouverture orale pour acquérir des données pour d'autres dents.

L'espaceur n'interdit pas le rapprochement ou l'écartement des mâchoires de l'utilisateur l'une de l'autre. Il autorise ainsi l'acquisition, notamment de photos représentant les dents de l'utilisateur, alors que l'utilisateur a la bouche ouverte ou la bouche fermée, en conservant les lèvres retroussées.

En faisant pivoter le kit autour de lui, l'utilisateur permet au téléphone portable d'observer des régions différentes de sa bouche, par exemple pour prendre des photos de la voute dentaire ou des dents du fond de la bouche.

De préférence, l'utilisateur prend des données 2D ou 3D pour au moins une vue de face, une vue de droite et une vue de gauche. De préférence, l'utilisateur prend des données 2D ou 3D pour au moins une vue bouche ouverte et au moins une vue bouche fermée, de préférence à chaque fois pour des vues de face, de droite et de gauche.

L'utilisateur retire ensuite l'espaceur 24 de sa bouche, et désassemble le téléphone portable. A cet effet, il lui suffit d'appuyer sur le premier bouton poussoir 74 pour dégager le premier cliquet 62 de la roue à rochet 64. Sous l'effet du ressort 56, le premier mors 52₁ s'écarte du deuxième mors et rejoint la position écartée, ce qui permet de dégager le téléphone portable 12.

L'utilisateur peut alors analyser les données acquises et/ou les transmettre avec le téléphone portable 12.

L'utilisateur sépare enfin l'espaceur 24 de l'adaptateur 26 en tirant suffisamment fort sur l'espaceur pour dégager les griffes 38.

Généralement, seul l'espaceur doit être nettoyé, voire stérilisé. Avantageusement, il ne comporte pas de partie mobile dont le fonctionnement risquerait d'être affecté par ces opérations.

En outre, il ne comporte pas d'arête rentrante susceptible de retenir des saletés. Le nettoyage ou la stérilisation en sont donc plus efficaces.

Bien entendu, l'ordre des opérations peut être modifié. Par exemple, l'utilisateur peut fixer l'espaceur sur l'adaptateur avant ou après la fixation du téléphone portable. Le démontage de l'espaceur peut être antérieur ou postérieur à celui du téléphone portable.

Comme cela apparaît clairement à présent, l'invention facilite considérablement l'acquisition de photos ou de modèles 3D, y compris représentant les faces vestibulaires de molaires, en particulier pour la mise en œuvre du procédé décrit dans PCT/EP2015/074896. Elle permet une acquisition rapide, dans d'excellentes conditions d'hygiène, typiquement en moins d'une minute, sans avoir recours à une personne spécialisée, notamment un dentiste ou un orthodontiste. L'acquisition peut être en particulier effectuée par l'utilisateur lui-même ou par un de ses proches, avec un simple téléphone portable, n'importe où, et en particulier en dehors d'un cabinet médical, dentaire ou d'orthodontie. En outre, l'acquisition est possible sans utilisation d'un outil prenant appui sur le sol pour immobiliser le téléphone portable, et notamment sans trépied.

Enfin, le kit permet une fixation rapide et fiable du téléphone portable, quelle que soit son épaisseur, sans risque de l'endommager.

Bien entendu, l'invention n'est pas limitée aux modes de réalisation décrits et représentés, fournis à des fins illustratives seulement.

Notamment, la forme de l'espaceur n'est pas limitative.

Le deuxième mors peut être mobile, et optionnellement monté sur l'embase comme le premier mors.

Dans un mode de réalisation, le premier mors 52₁ est monté mobile sur l'embase 50 de manière à se rapprocher élastiquement du deuxième mors. Le premier système anti-retour peut être alors conçu pour empêcher ce rapprochement, sa désactivation provoquant un retour élastique vers une position rapprochée.

## Revendications

1. Kit d'acquisition comportant :
- un téléphone portable (12) comportant
- une face avant (12₁),
- une face arrière (12₂) présentant un écran (20), et
- un chant (22), définissant l'épaisseur du téléphone portable et joignant les faces avant et arrière ;
le téléphone portable étant muni d'un objectif (16) d'acquisition de données 2D et/ou 3D ;
- un support (14) de téléphone portable comportant
- un espaceur (24) débouchant par une ouverture orale (Oo) destinée à être introduite dans la bouche de l'utilisateur et par une ouverture d'acquisition (Oa'), et
- un adaptateur (26) fixé sur l'espaceur (24) et définissant avec l'espaceur (24) une chambre (28) débouchant par l'ouverture orale (Oo) et par une ouverture d'adaptateur (Oa), l'adaptateur comportant une embase (50) sur laquelle le téléphone portable est rigidement fixé, de manière amovible, dans une position d'acquisition dans laquelle l'objectif (16) a une vue au moins partielle de l'ouverture orale (Oo), à travers l'ouverture d'adaptateur (Oa) et l'ouverture d'acquisition (Oa'), l'adaptateur (26) comportant des premier et deuxième mors (52₁ ; 52₂) entre lesquels le téléphone portable est serré, suivant une direction de serrage (Z₅₂).

2. Kit d'acquisition selon la revendication immédiatement précédente, dans lequel l'adaptateur (26) étant dépourvu de butée limitant le déplacement perpendiculairement à la direction de serrage.

3. Kit d'acquisition selon l'une quelconque des revendications précédentes, dans lequel le premier mors (52₁) et/ou le deuxième mors (52₂) présente(nt) la forme d'un berceau recevant le chant (22) du téléphone portable (12).

4. Kit d'acquisition selon la revendication immédiatement précédente, dans lequel le premier mors (52₁) et/ou le deuxième mors (52₂) est mobile sur l'embase (50) de manière incrémentielle.

5. Kit d'acquisition selon la revendication immédiatement précédente, dans lequel l'adaptateur (26) comporte :
- un premier système anti-retour (62,64,66) désactivable empêchant un mouvement du premier mors (52₁) à l'écart du téléphone portable (12), dit « mouvement d'écartement », et
- un organe élastique (56) agissant sur le premier mors (52₁) de manière à produire un dit mouvement d'écartement.

6. Kit d'acquisition selon la revendication immédiatement précédente, dans lequel le premier système anti-retour (62,64,66) comporte :
- une roue à rochet (64) mécaniquement couplée au premier mors (52₁) de manière qu'une rotation de la roue à rochet (64) entraine un déplacement du premier mors (52₁) par rapport à l'embase (50),
- un premier cliquet (62) désactivable, engagé dans la roue à rochet, et
- un premier organe élastique (66) poussant élastiquement le premier cliquet 62 contre la roue à rochet (64).

7. Kit d'acquisition selon la revendication immédiatement précédente, dans lequel
le premier mors comporte une première crémaillère (68), et
l'adaptateur (26) comporte une roue étagée (70) montée à rotation sur l'embase (50), et comportant
- la roue à rochet (64), et
- un pignon (72), coaxial à la roue à rochet (64), coopérant avec la première crémaillère (68) de manière qu'une rotation du pignon (72) entraine le premier mors.

8. Kit d'acquisition selon l'une quelconque des deux revendications immédiatement précédentes, dans lequel le premier mors (52₁) et/ou le deuxième mors (52₂) comporte(nt) un tampon (71) élastiquement déformé et en appui sur le téléphone portable, la déformabilité du tampon autorisant au moins un passage entre deux positions indexées consécutives définies par la coopération de la roue à rochet (64) et du premier cliquet (62).

9. Kit d'acquisition selon l'une quelconque des revendications précédentes, dans lequel l'espaceur (24) présente une forme générale tubulaire.

10. Kit d'acquisition selon l'une quelconque des revendications précédentes, dans lequel la direction de serrage (Z₅₂) est perpendiculaire à la direction de la longueur (L₁₂) du téléphone portable (12) et/ou perpendiculaire à la direction de la largeur (l₁₂) du téléphone portable (12) et/ou suivant une diagonale joignant deux coins du téléphone portable.
